# EUROPEAN PATENT SPECIFICATION

(11) **EP 1 455 281 B1**
(45) Date of publication and mention of the grant of the patent: **30.07.2008**
(21) Application number: 02788781.9
(22) Date of filing: 10.12.2002
(51) Int. Cl.: G06F 19/00, G06F 17/30

(54) **APPARATUS FOR PREDICTING STEREOSTRUCTURE OF PROTEIN AND PREDICTION METHOD**
VORRICHTUNG ZUM VORHERSAGEN DER STEREOSTRUKTUR EINES PROTEINS UND VORHERSAGEVERFAHREN
APPAREIL DE PREDICTION DE LA STRUCTURE TRIDIMENSIONNELLE D'UNE PROTEINE ET PROCEDE DE PREDICTION ASSOCIE

(30) Priority: 10.12.2001 JP 2001375857
(43) Date of publication of application: 08.09.2004
(73) Proprietor: FUJITSU LIMITED, Kawasaki-shi, Kanagawa 211-8588 (JP)
(72) Inventor: SAKAI, K., FUJITSU KYUSHU SYSTEM ENGINEERINGLTD., Fukuoka-shi, Fukuoka 814-8589 (JP)
(74) Representative: Sunderland, James Harry
(86) International application number: PCT/JP2002/012942
(87) International publication number: WO 2003/054743

(56) References cited:
- RUSSELL ROBERT B ET AL: "Protein fold recognition by mapping predicted secondary structures" JOURNAL OF MOLECULAR BIOLOGY, vol. 259, no. 3, 1996, pages 349-365, XP002435130 ISSN: 0022-2836
- DI FRANCESCO V ET AL: "FORESST: Fold recognition from secondary structure predictions of proteins" BIOINFORMATICS (OXFORD), vol. 15, no. 2, February 1999 (1999-02), pages 131-140, XP002435131 ISSN: 1367-4803
- GURUPRASAD K. ET AL.: 'Analysis of gammabeta, betagamma, gammagamma, betabeta multiple turns in proteins' THE JOURNAL OF PEPTIDE RESEARCH vol. 56, no. 4, October 2000, pages 250 - 263, XP002962397
- CHOU K.-C.: 'Prediction of tight turns and their types in proteins' ANALYTICAL BIOCHEMISTRY vol. 286, no. 1, 01 November 2000, pages 1 - 16, XP002962398

## Description

### Technical Field

The present invention relates to an apparatus and a method for predicting a three-dimensional structure of a protein, and in particular to an apparatus and a method for predicting a three-dimensional structure of a protein formed of amino acids, based on an amino-acid sequence.

### Background Art

Proteins are polypeptides in which twenty kinds of amino acids are peptide bonded. In organisms and the like, normally, polypeptides are ingeniously folded, and complex three-dimensional structures of the proteins are realized by folding polypeptides, which originally have a form of a straight chain.

A sequence of amino acids realizing a straight-chain polypeptide is called a primary sequence of a protein. The primary sequence of a protein indicates which amino acids are arranged in what order in the protein. That is, the kinds and order of amino acid residues forming a protein are determined by the primary sequence of the protein.

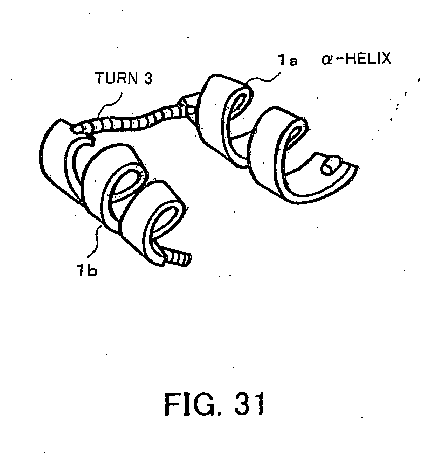

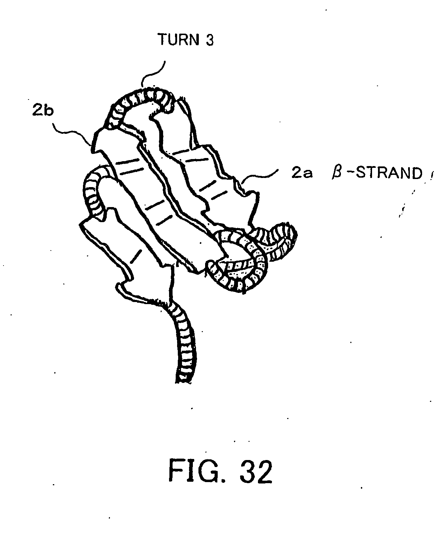

In each polypeptide chain, amino acid residues close to each other are hydrogen bonded to form certain stable structures called secondary structures, which include α-helices, β-sheets, and turns. Hereinbelow, the secondary structures are explained with reference to drawings. FIG. 31 is a diagram illustrating α-helices, and FIG. 32 is a diagram illustrating β-sheets. As illustrated by the references 1a and 1b in FIG. 31, the α-helices each have a helical structure. That is, the name "α-helix" is derived from its structure. As illustrated by the references 2a and 2b in FIG. 32, two or more chains called β-strands are arranged side by side so as to have the appearance of a sheet (or paper or plane). The portions connecting the α-helices and the β-sheets are called turns. In the example of FIG. 31, the turn 3 connects the α-helices 1a and 1b. In the example of FIG. 32, the turn 3 connects the β-sheets 2a and 2b.

When secondary structures as above are complexly combined, and the entire polypeptide is folded, an overall structure unique to a protein is finally obtained. The finally obtained structure is called a tertiary structure. The tertiary structure is hereinafter referred to as a three-dimensional structure. Thus, local secondary structures such as α-helices and β-sheets are connected through turns so as to form various three-dimensional structures which are bent at the turns. In the example of FIG. 31, the α-helices a1 and 1b are connected through the turn 3 which is bent 180 degrees, so that the α-helices a1 and 1b are arranged antiparallel to each other.

Incidentally, the three-dimensional structure of each protein is closely related to whether or not the protein expresses the functions which the protein has. Therefore, in order to understand the functions of each protein, it is important to recognize the three-dimensional structure of the protein. Thus, in the fields in which proteins are utilized and studied, such as pharmacology or biochemistry, the three-dimensional structures of the proteins have been analyzed by using the X-ray diffraction and NMR (Nuclear Magnetic' Resonance) techniques. However, these analyzing techniques require much time and entail enormous cost.

On the other hand, since a number of three-dimensional structures have been determined with improvements in the above analyzing techniques, and proteins having similar structures have been discovered, various methods for predicting the three-dimensional structures of proteins based on information on known three-dimensional structures have been developed. In such circumstances, a method called homology modeling is currently receiving special attention. For example, see Lee, R., "Protein model Building Using Structural Homology," Nature 356 (1992) pp. 543-544. According to the homology modeling method, analysis is performed based on an assumption that the three-dimensional structures of proteins are similar when the primary sequences of the proteins are similar. Specifically, first, a three-dimensional-structure database in which proteins the three-dimensional structures of which are known are registered is searched. Then, when an amino-acid sequence of a protein the structure of which is required to be predicted has a similarity to an amino-acid sequence of one of the registered proteins having known three-dimensional structures, the three-dimensional structure of the one of the registered proteins is acquired. Finally, the required three-dimensional structure corresponding to the amino-acid sequence of the desired protein is predicted by modeling based on the acquired three-dimensional structure.

However, according to the technique of predicting a three-dimensional structure of a protein using the conventional homology modeling method, it is impossible to predict a three-dimensional structure of a protein which does not have an amino-acid sequence similar to that of a protein having a known three-dimensional structure.

As described above, according to the homology modeling method, an unknown three-dimensional structure of a protein is predicted based on the consideration that the three-dimensional structures of proteins will be similar when the primary sequences of the proteins are similar. Therefore, it is impossible to predict the three-dimensional structure of a protein when there is no protein which has a known three-dimensional structure and a similar amino-acid sequence. For example, when a new amino-acid sequence is discovered, it is impossible to predict a three-dimensional structure of a protein having the new amino-acid sequence.

In recent years, accuracy of prediction of secondary structures constituting proteins has been improved. When a three-dimensional structure is predicted based on a precise prediction of the secondary structures, α-helices and β-strands can be determined by their own structures. However, when a β-sheet is formed of β-strands, or when α-helices are connected, or when an α-helix and a β-sheet are connected, it is necessary to use the structures called turns in the portions of the three-dimensional structure at which the secondary structures are connected. Nevertheless, the degrees of freedom in the definitions of the turns are high, and it is very difficult to determine the turns. For this reason, the currently available modeling programs for predicting three-dimensional structures of proteins have not yet succeeded in determining turns or reproducing the structures of the turns.

### Disclosure of the Invention

The present invention is made in view of the above problems, and the object of the present invention is to provide a protein three-dimensional-structure prediction apparatus and a protein three-dimensional-structure prediction method for predicting a three-dimensional structure of a protein by determining turns.

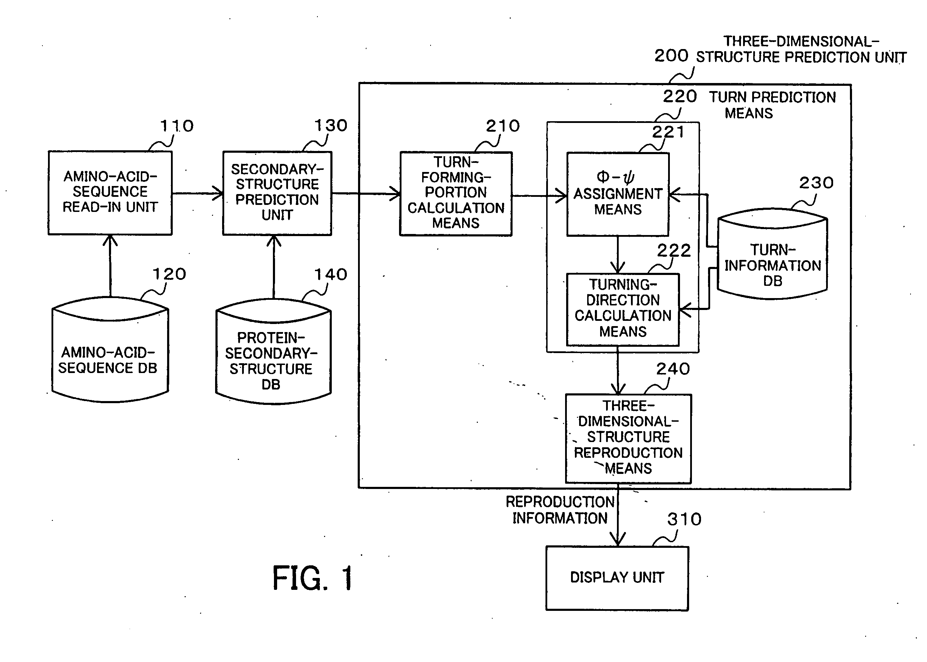

In order to solve the aforementioned problem, a protein three-dimensional-structure prediction apparatus as illustrated in FIG. 1 is provided. In the protein three-dimensional-structure prediction apparatus according to the present invention, an amino-acid sequence as a primary sequence of a protein is read in, and the amino-acid sequence and local secondary-structure information which is predicted based on the amino-acid sequence are inputted into a three-dimensional-structure prediction unit 200. In the three-dimensional-structure prediction unit 200, a turn-forming-portion calculation means 210 extracts a sequence of amino acids which form a turn, calculates the number of amino acids included in the sequence of the amino acids which form the turn, and sends the number to a turn prediction means 220 together with the secondary-structure information. The turn prediction means 220 acquires turn-structure information on a probable turn, which is obtained according to the calculated number of the amino acids forming the turn and the secondary-structure information, and reproduces a turning portion based on the turn-structure information. It is found that the structures of turns can be classified into a plurality of patterns according to the secondary structures and values of the number of amino acids forming a turn. The turn-structure information is information on the structure of a probable turn, and is obtained for each classification. Therefore, it is possible to reproduce the probable turn by using the turn-structure information. A three-dimensional-structure reproduction means 240 reproduces the entire three-dimensional structure of the protein by using the reproduced turning portion, and generates reproduction information in a predetermined form.

In addition, in order to solve the aforementioned problem, a protein three-dimensional-structure prediction method is provided. In the protein three-dimensional-structure prediction method according to the present invention, turn-structure information on probable turns is acquired in advance and stored in a predetermined storage means, in correspondence with secondary-structure information and values of the number of amino acids forming a turn, where the turn-structure information is extracted from three-dimensional-structure information for proteins having known three-dimensional structures. When an amino-acid sequence of a desired protein and secondary-structure information corresponding to the amino-acid sequence are acquired, the number of amino acids forming a turn is calculated based on the secondary-structure information. Then, the turn-structure information corresponding to the secondary-structure information and the number of the amino acids forming the turn is extracted by searching for the turn-structure information based on the secondary-structure information and the number of the amino acids forming the turn, and reproduction information in which the entire three-dimensional structure of the protein is reproduced is generated by using a turning portion reproduced based on the extracted turn-structure information.

The above and other objects, features and advantages of the present invention will become apparent from the following description when taken in conjunction with the accompanying drawings which illustrate preferred embodiment of the present invention by way of example.

### BRIEF DESCRIPTION OF THE DRAWINGS

FIG. 1 is a diagram illustrating a construction of a protein three-dimensional-structure prediction apparatus according to an embodiment of the present invention.
FIG. 2 is a diagram illustrating a polypeptide.
FIG. 3 is a diagram illustrating parameters for determining a three-dimensional structure of a protein.
FIG. 4 is a diagram illustrating an example of an amino-acid sequence and an example of secondary-structure information corresponding to the amino-acid sequence.
FIG. 5 is a diagram illustrating an example of turn-structure information.
FIG. 6 is a diagram illustrating examples of patterns of dihedral angles registered in the turn-structure information.
FIG. 7 is a flow diagram illustrating an entire sequence of processing for predicting a three-dimensional structure of a protein according to an embodiment of the present invention.
FIG. 8 is a flow diagram illustrating a sequence of processing for reproducing a turn.
FIG. 9 is a flow diagram illustrating a sequence of processing for reproducing a turn in a β-strand structure.
FIG. 10 is a diagram illustrating an example of display of a three-dimensional structure of a protein realized by the protein three-dimensional-structure prediction apparatus and the protein three-dimensional-structure prediction method according to an embodiment of the present invention.
FIG. 11 is a flow diagram illustrating a sequence of processing for reproducing a turn according to another embodiment of the present invention.
FIG. 12 is a diagram illustrating classification into different types of turns used in three-dimensional structural analysis of proteins according to the present invention.
FIG. 13 is a Ramanchandran plot of the first residues in 180-degree β-strand turns.
FIG. 14 is a Ramanchandran plot of the second residues in the 180-degree β -strand turns.
FIG. 15 is a graph indicating distributions of the angles φ and ψ in the first residues in 180-degree β-strand turns.
FIG. 16 is a graph indicating distributions of the angles φ and ψ in the second residues in the 180-degree β-strand turns.
FIG. 17 is a diagram indicating representative values of the dihedral angles φ and ψ in and corresponding turning directions of 180-degree β-strand turns, which are obtained by three-dimensional structural analysis.
FIG. 18 is a diagram illustrating an example of display of a first three-dimensional structure of a 180-degree β-strand turn.
FIG. 19 is a diagram illustrating an example of display of a second three-dimensional structure of a 180-degree β-strand turn.
FIG. 20 is a graph indicating distributions of the angles φ and ψ in the first residues in 90-degree turns.
FIG. 21 is a diagram indicating representative values of the dihedral angles φ and ψ in and corresponding turning directions of 90-degree β-strand turns, which are obtained by three-dimensional structural analysis.
FIG. 22 is a diagram illustrating an example of display of a first three-dimensional structure of a 90-degree β -strand turn.
FIG. 23 is a diagram illustrating an example of display of a second three-dimensional structure of a 90-degree β-strand turn.
FIG. 24 is a graph indicating distributions of the angles φ and ψ in the first residues in 60-degree β-strand turns.
FIG. 25 are probable values of the dihedral angles φ and ψ in 60-degree β -strand turns, which are obtained by three-dimensional structural analysis.
Fig. 26 is a diagram illustrating an example of display of a first three-dimensional structure of a 60-degree β-strand turn.
FIG. 27 is a diagram illustrating an example of display of a second three-dimensional structure of a 60-degree β -strand turn.
FIG. 28 are probable values of the dihedral angles and ψ in structures obtained by three-dimensional structural analysis in accordance with the straight-chain model.
FIG. 29 is a diagram illustrating an example of display of a first three-dimensional structure of a turn in an α-helix structure.
FIG. 30 is a diagram illustrating an example of display of a second three-dimensional structure of a turn in an α-helix structure.
FIG. 31 is a diagram illustrating α-helices.
FIG. 32 is a diagram illustrating β-sheets.

### Best Mode for Carrying out the Invention

Embodiments of the present invention are explained below with reference to drawings.

FIG. 1 is a diagram illustrating a construction of a protein three-dimensional-structure prediction apparatus according to an embodiment of the present invention.

The protein three-dimensional-structure prediction apparatus according to the present invention comprises an amino-acid-sequence read-in unit 110 for reading in an amino-acid sequence of a protein as a primary sequence of the protein, an amino-acid-sequence database (amino-acid-sequence DB) 120 for storing amino-acid sequences, a secondary-structure prediction unit 130 for predicting secondary structures based on an amino-acid sequence as a primary sequence, a protein-secondary-structure database (protein-secondary-structure DB) 140 for storing information on secondary structures constituting proteins, a three-dimensional-structure prediction unit 200 for predicting a three-dimensional structure of a protein, and a display unit 310 for displaying a reproduced three-dimensional structure.

Specifically, the amino-acid-sequence read-in unit 110 reads in from the amino-acid-sequence DB 120 or the like an amino-acid sequence of a protein as a primary sequence of amino acids forming a protein. At this time, the amino-acid sequence can be read in any manner. For example, the desired amino-acid sequence can be downloaded through a communication network to which the amino-acid-sequence DB 120 is connected. Alternatively, a user can input the amino-acid sequence from an input device such as a keyboard, or the amino-acid sequence may be recorded in advance in a recording medium such as a flexible disk so that the protein three-dimensional-structure prediction apparatus of FIG. 1 can read in the amino-acid sequence from the recording medium.

The amino-acid-sequence DB 120 is a database in which amino-acid sequences of proteins are stored and managed. It is possible to acquire a desired amino-acid sequence from the amino-acid-sequence DB 120 by searching the amino-acid-sequence DB 120 by a keyword or the like. The amino-acid-sequence DB 120 may be arranged either inside or outside the protein three-dimensional-structure prediction apparatus. In the latter case, the amino-acid-sequence DB 120 is connected to the protein three-dimensional-structure prediction apparatus through a communication line. Further, the amino-acid-sequence DB 120 may be a data bank in a public organization or the like which stores and manages registered amino-acid sequences.

The secondary-structure prediction unit 130 acquires secondary-structure information which indicates predictions of secondary structures corresponding to the amino-acid sequence read in by the amino-acid-sequence read-in unit 110. For example, the secondary-structure prediction unit 130 searches the protein-secondary-structure DB 140 in which secondary structures constituting proteins are registered, and reads out secondary-structure information corresponding to the primary sequence. Alternatively, the secondary-structure prediction unit 130 may generate the secondary-structure information by predicting the secondary structures by use of the Garnier-Robson method or the like. The secondary-structure information is sent to the three-dimensional-structure prediction unit 200 together with information on the amino-acid sequence (amino-acid-sequence information).

The protein-secondary-structure DB 140 is a database in which secondary-structure information indicating predictions of secondary structures constituting proteins is recorded. It is possible to acquire secondary-structure information for a desired protein from the protein-secondary-structure DB 140 by searching the protein-secondary-structure DB 140 by a keyword or the like. As in the case of the amino-acid-sequence DB 120, the protein-secondary-structure DB 140 can be realized in an arbitrary manner.

The three-dimensional-structure prediction unit 200 has a function of predicting a three-dimensional structure of a protein based on the amino-acid-sequence information and the secondary-structure information, and generates reproduction information for reproducing the predicted three-dimensional structure. The reproduction information can be outputted in any form adapted for a use of the reproduction information. For example, when the reproduction information is outputted to the display unit 310, the reproduction information has a form adapted for display, e.g., the reproduction information may be represented by coordinates or the like for displaying the predicted three-dimensional structure. Or when the reproduction information is utilized as an input structure in a molecular-dynamics simulation or a simulation of molecular behavior in accordance with the molecular orbital method, the reproduction information is outputted in a form adapted for simulation software used for the simulation. The processing for predicting a three-dimensional structure is explained later.

The display unit 310 three-dimensionally displays the three-dimensional structure of the protein based on the reproduction information.

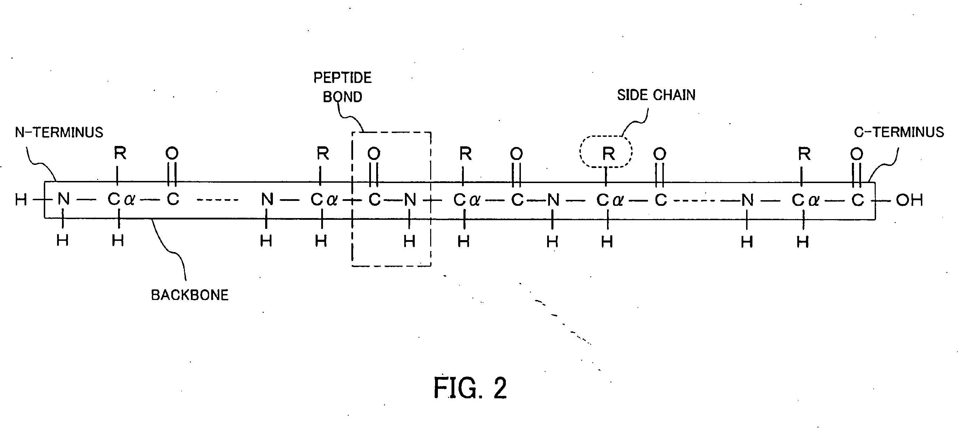

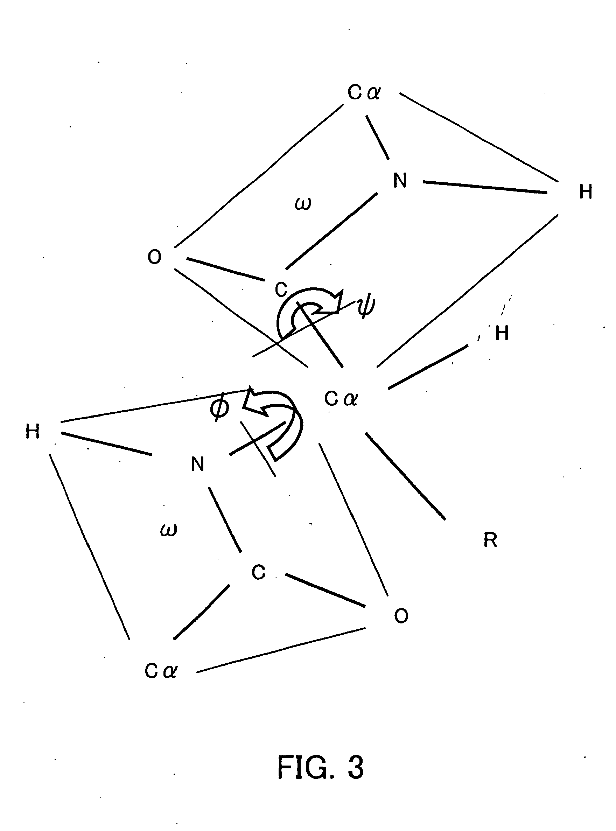

Before explaining the three-dimensional-structure prediction unit 200 in detail, display of a protein and parameters for determining a three-dimensional structure of a protein are explained with reference to drawings. FIG. 2 is a diagram illustrating a polypeptide, and FIG. 3 is a diagram illustrating parameters for determining a three-dimensional structure of a protein. In each amino acid, a carboxyl group (-COOH) and an amino group (-NH₂) are bonded to a carbon atom Cα. The bond (-CO-NH-) illustrated in FIG. 2, which is realized by removing H₂ from the amino group and the carboxyl group, is called a peptide bond. The polypeptide is formed by bonding a great number of amino acids by peptide bonds. In FIG. 2, the portions indicated by R are called side chains, and the portion other than the side chains is called a principal chain (or a main chain, or a backbone). In the drawings which illustrate a three-dimensional structure of a protein, the principal chains as illustrated in FIG. 2 are indicated by the shapes of ribbons, strings, or the like. In addition, in each β-strand, the direction from the N-terminus to the C-terminus is indicated by an arrow. Incidentally, six atoms constituting the principal chain of the polypeptide are located in an identical plane as illustrated in FIG. 3. Therefore, the three-dimensional structure of each protein is determined by conformational angles in the two bonds N-Cα and Cα-O in each amino acid. The two conformational angles (dihedral angles) are indicated by phi (φ) and psi (ψ).

Referring back to FIG. 1, the three-dimensional-structure prediction unit 200 is explained below. The three-dimensional-structure prediction unit 200 comprises a turn-forming-portion calculation means 210 for calculating the number of amino acids forming a turn, a turn prediction means 220 including an φ-ψ assignment means 221 and a turning-direction calculation means 222, a turn-information database (turn-information DB) 230 in which turn-structure information on structures of turns is recorded, and a three-dimensional-structure reproduction means 240 for reproducing a three-dimensional structure, where the φ-ψ assignment means 221 is a dihedral-angle assignment means provided for assignment of the dihedral angles φ and ψ, and the turning-direction calculation means 222 is provided for calculating a turning direction.

Specifically, based on the aforementioned secondary structures, the turn-forming-portion calculation means 210 extracts an amino-acid sequence of a turn-forming portion at which the turn is formed, and calculates the number of amino acids in the extracted amino-acid sequence. Hereinafter, the numbers of amino acids are referred to as the numbers of residues.

The turn prediction means 220 reproduces the turn based on the secondary-structure information and the number of residues forming the turn which is calculated by the turn-forming-portion calculation means 210.

The φ-ψ assignment means 221 in the turn prediction means 220 acquires turn-structure information which indicates a structure of a probable turn registered in the turn-information DB 230, based on the number of residues calculated by the turn-forming-portion calculation means 210, and assigns the dihedral angles φ and ψ based on the acquired turn-structure information.

The turning-direction calculation means 222 in the turn prediction means 220 searches the turn-information DB 230 for a turning direction which is determined in correspondence with the turn and the secondary structures on both sides of the turn, and determines the turning direction based on the search result. Hereinafter, the turning direction is indicated to be right or left. In each left turn, when the side chain of the carbon atom Cα in the first residue in the left turn is located in the direction of 90 degrees (with respect to the direction of the backbone) from the carbon atom Cα in the first residue, the side chain of the carbon atom Cα in the last residue in the left turn is located in the direction of 270 degrees from the carbon atom Cα in the first residue. In each right turn, when the side chain of the carbon atom Cα in the first residue in the right turn is located in the direction of 90 degrees (with respect to the direction of the backbone) from the carbon atom Cα in the first residue, the side chain of the carbon atom Cα in the last residue in the right turn is located in the direction of 90 degrees from the carbon atom Cα in the first residue.

In the turn-information DB 230, turn-structure information indicating structures of probable turns which are obtained for the secondary structures and values of the number of amino acids forming a turn is stored in association with the secondary structures and the number of residues forming the turn. As explained before, the structure of the polypeptide is ruled by the two parameters (φ and ψ) in each amino acid. The present inventor has determined probable values of the angles φ and ψ for each residue constituting each turn which appears between β-strands, by analyzing known three-dimensional structures of proteins. In addition, the present inventor has found that the turning direction of each turn can be classified into left or right according to whether the number of residues preceding the turn and forming a β-strand is odd or even, respectively. Further, the present inventor has also found that in the case where a turning portion is inserted between α-helices, the turning portion can be classified into a 180-degree turn which orients the α-helices in opposite directions or a turn which orients the α-helices in an identical direction, according to whether the number of residues forming the turning portion inserted between the α-helices is odd or even, respectively. Thus, based on the above findings, it is possible to produce turn-structure information indicating structures (e.g., the angles φ and ψ , the turning directions, and the like) of probable turns which are obtained in correspondence with the secondary structures and values of the number of residues forming a turn. Since the turn-structure information can be obtained by analyzing known three-dimensional structures of proteins, it is preferable to produce turn-structure information in advance, and register the produced turn-structure information in a database. In this example, the turn-structure information produced as above is stored and managed in the turn-information DB 230.

The three-dimensional-structure reproduction means 240 reproduces a three-dimensional structure of a protein based on a reproduced turn and the secondary-structure information, and generates reproduction information in a predetermined form. The reproduction information can be outputted in an arbitrary form adapted for a use of the reproduction information. In this case, the reproduction information is generated for the display unit 310 in such a manner that the three-dimensional structure is represented by three-dimensional coordinates.

Next, operations of the protein three-dimensional-structure prediction apparatus having the above construction are explained below.

The amino-acid-sequence read-in unit 110 reads in from the amino-acid-sequence DB 120 an amino-acid sequence of a protein of which the three-dimensional structure is to be predicted, and sends the amino-acid sequence to the secondary-structure prediction unit 130. The secondary-structure prediction unit 130 determines whether or not secondary structures corresponding to the amino-acid sequence read in as above are stored in the protein-secondary-structure DB 140, by searching the protein-secondary-structure DB 140. When the secondary structures corresponding to the amino-acid sequence are stored in the protein-secondary-structure DB 140, the secondary-structure prediction unit 130 reads in the secondary structures corresponding to the amino-acid sequence. When the secondary structures corresponding to the amino-acid sequence are not stored in the protein-secondary-structure DB 140, or when necessary, the secondary-structure prediction unit 130 predicts secondary structures corresponding to the amino-acid sequence by using the Garnier-Robson method or the like. Then, secondary-structure information indicating the secondary structures is sent to the three-dimensional-structure prediction unit 200.

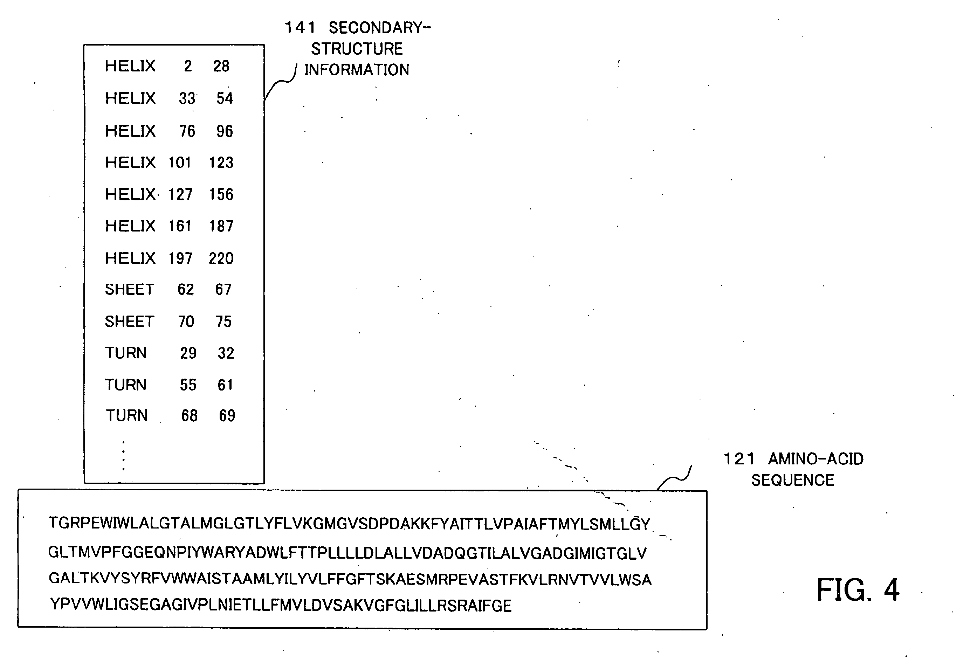

Concrete examples of the amino-acid sequences stored in the amino-acid-sequence DB 120 and the secondary-structure information stored in the protein-secondary-structure DB 140 are explained below. FIG. 4 is a diagram illustrating an example of an amino-acid sequence and an example of secondary-structure information corresponding to the amino-acid sequence. Although the examples of FIG. 4 are written in a PDB format, the amino-acid sequences and secondary-structure information may be written in any other format. For example, the amino-acid sequences and secondary-structure information may be written side by side. The amino-acid sequence 121 in FIG. 4 is a sequence of amino acids, where each alphabetical character indicates a kind of amino acid. In addition, the secondary-structure information 141 indicates secondary structures corresponding to the amino-acid sequence 121, where the numbers following "HELIX," "SHEET," and "TURN" indicate the locations at which the corresponding α-helices, β-sheets, and turns appear in the amino-acid sequence 121, respectively.

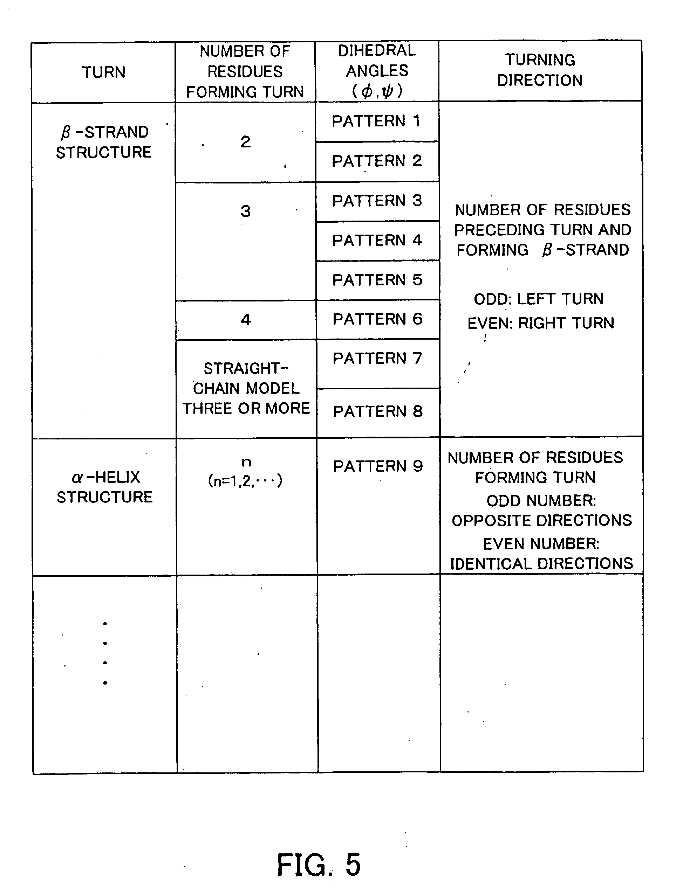

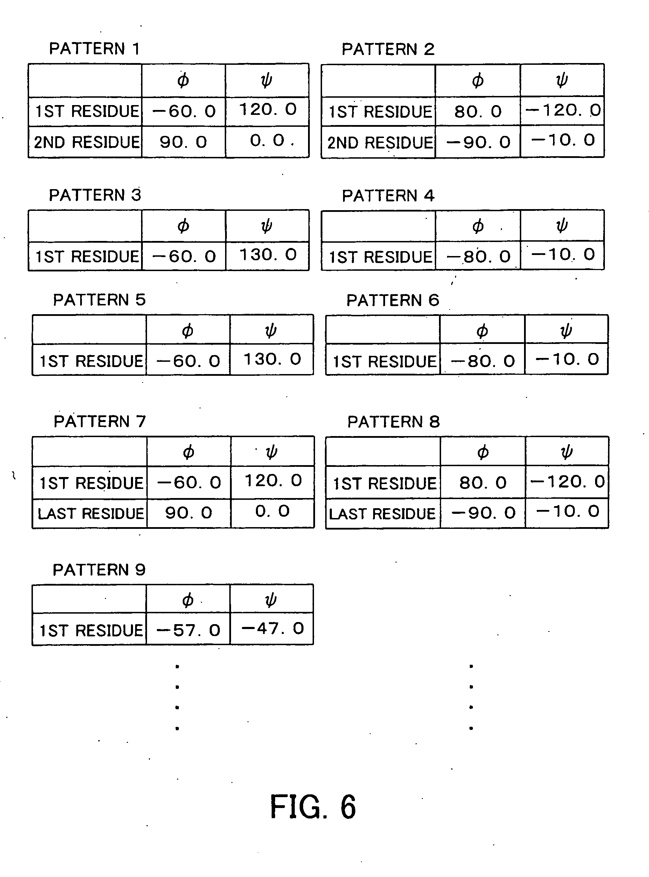

In the three-dimensional-structure prediction unit 200, the turn-forming-portion calculation means 210 calculates the number of residues constituting a turn-forming portion which forms each turn, and the turn prediction means 220 reproduces the turn by using the number of residues and the secondary-structure information. In the turn-information DB 230, the turn-structure information indicating structures of probable turns which are obtained for secondary structures and values of the number of residues is registered in advance. FIG. 5 is a diagram illustrating an example of turn-structure information, and FIG. 6 is a diagram illustrating examples of patterns of dihedral angles registered in the turn-structure information.

As illustrated in FIG. 5, the dihedral angles and ψ formed in a turn and a rule of the turning direction are registered as the turn-structure information for each location at which a turn appears. Hereinafter, turns appearing between β-strands are referred to as turns in β-strand structures, and turns appearing between α-helices are referred to as turns in α-helix structures. Patterns of the dihedral angles which can be selected according to values of the number of residues forming a turn are predetermined.

According to an embodiment of the present invention, the angles φ and ψ obtained by three-dimensional structural analysis (explained later) are registered in accordance with values of the number of residues forming a 180-degree turn which turns the direction of a strand structure to the opposite direction. The patterns 1 and 2 each include the most probable values of the angles φ and ψ in a 180-degree turn which is formed of two residues. The patterns 3, 4, and 5 each include the most probable values of the angles φ and ψ in a 180-degree turn which is formed of three residues, where a 90-degree turn occurs between the three residues. In addition, the pattern 6 includes the most probable values of the angles φ and ψ in a 180-degree turn which is formed of four residues, where a 60-degree turn occurs between the four residues. Alternatively, it is possible to consider a model of a 180-degree turn in which a 90-degree turn occurs at each of the first and last ones of residues forming the 180-degree turn, and the remaining residues between the first and last residues are bonded so as to form a straight chain. Hereinafter, this model is referred to as a straight-chain model. The patterns 7 and 8 each include the most probable values of the angles φ and ψ in a 180-degree turn realized based on the straight-chain model.

On the other hand, it is prescribed that the dihedral angles φ and ψ in each turn in an α-helix structure have predetermined values indicated in the pattern 9 regardless of the number of residues forming each turn. For example, values of the angles φ and ψ in the respective patterns are registered as illustrated in FIG. 6.

On the other hand, it is prescribed that the turning direction of each turn in a β-strand structure is left when the number of residues preceding the turn and forming a β-strand is odd, and a right turn when the number of residues preceding the turn and forming a β-strand is even. It is also prescribed that in each turn in an α-helix structure, α-helices on the both sides of the turn are oriented in opposite directions when the number of residues forming the turn inserted between the α-helices is odd, and in an identical direction when the number of residues forming the turn inserted between the α-helices is even.

As explained above, it is possible to extract from the turn-structure information the prescribed information indicating the dihedral angles and the turning direction, based on the secondary-structure information. Although not shown, turn-structure information for other turns such as turns each appearing between an α-helix and a β-strand is also registered.

The φ-ψ assignment means 221 in the turn prediction means 220 searches the turn-information DB 230 based on the secondary-structure information and the number of residues, and acquires probable values of the dihedral angles. Similarly, the turning-direction calculation means 222 searches the turn-information DB 230 based on the secondary-structure information and the number of residues, and acquires the turning direction.

For example, in the case of a turn in a β-strand structure, the angles φ and ψ are determined according to the number of residues forming the turn, and the turning direction is predetermined to be left or right according to the number of residues preceding the turn and forming a β-strand. In addition, in the case of a turn in an α - helix structure, the angles φ and ψ are predetermined, and α-helices on the both sides of the turn are determined to be oriented in opposite directions (i.e., the turn between the α-helices is determined to be a 180-degree turn) when the number of residues forming the turn inserted between the α-helices is odd, or in an identical direction when the number of residues forming the turn inserted between the α-helices is even. Similarly, in the case of a turn between an α - helix and a β-sheet, the angles φ and ψ and the turning direction are determined. Thus, the structure of each turn can be determined based on the information in the turn-information DB 230. The three-dimensional-structure reproduction means 240 reproduces a three-dimensional structure based on the secondary-structure information and turns determined as above, and generates reproduction information in a predetermined form.

As explained above, it is possible to predict a three-dimensional structure of a protein by reproducing turns. In particular, as distinct from the homology modeling method, unknown three-dimensional structures of proteins can be predicted according to the present invention. The predicted three-dimensional structures can be utilized as input structures in molecular-dynamics simulations or simulations of molecular behavior in accordance with the molecular orbital method.

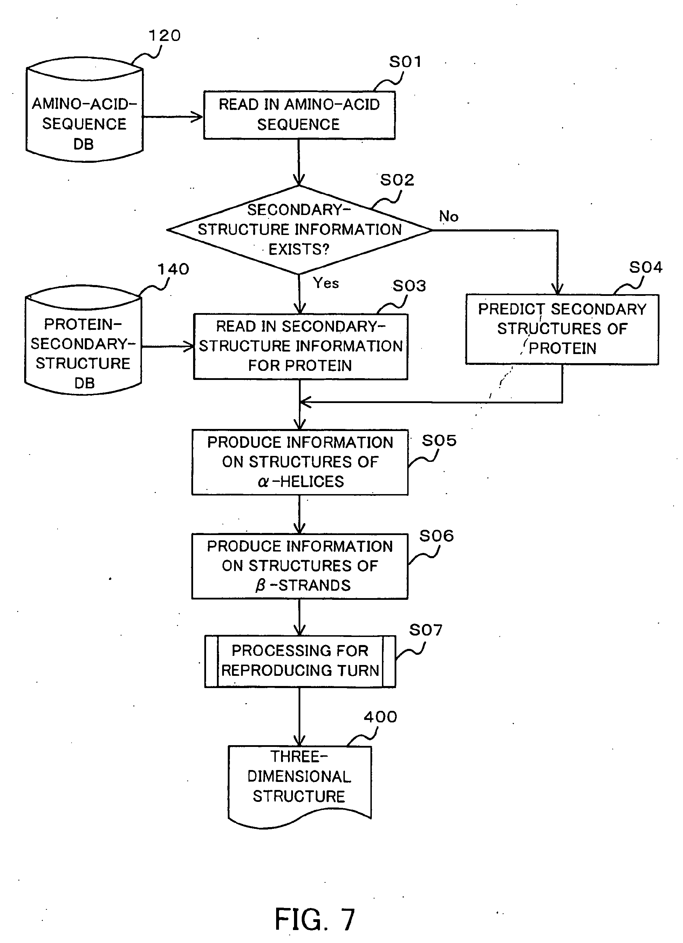

Next, the protein three-dimensional-structure prediction method according to the present invention is explained below. FIG. 7 is a flow diagram illustrating an entire sequence of processing for predicting a three-dimensional structure of a protein according to an embodiment of the present invention.
[Step S01] First, an amino-acid sequence as a primary sequence, for example, the amino-acid sequence 121 in FIG. 4, is read in from the amino-acid-sequence DB 120.
[Step S02] Next, it is determined whether or not secondary-structure information corresponding to the amino-acid sequence exists in the protein-secondary-structure DB 140. When yes is determined, the operation goes to step S03. When no is determined, the operation goes to step S04.
[Step S03] In the case where the secondary-structure information exists in the protein-secondary-structure DB 140, information on secondary structures corresponding to the protein, for example, the secondary-structure information 141 in FIG. 4, is read in from this database.
[Step S04] In the case where the secondary-structure information does not exist, secondary structures corresponding to the protein are predicted by the Garnier-Robson method or the like, and secondary-structure information is generated.
[Step S05] Structure information on structures of α-helices is generated based on the above secondary-structure information.
[Step S06] Similarly, structure information on structures of β -strands is generated.
   Through the above steps, it is possible to finally generate three-dimensional-coordinate information which is necessary for reproduction of three-dimensional structures of the α-helices and the β-strands. For this purpose, well known procedures can be used.
[Step S07] Next, processing for reproducing turns is performed based on the secondary-structure information. Although details of the processing for reproducing turns are explained later, three-dimensional-coordinate information which is necessary for reproduction of three-dimensional structures is generated by the processing for reproducing turns.

When the above sequence of processing is executed, the three-dimensional-coordinate information for the α-helices, β-strands, and turns is generated through the above steps, and three-dimensional-structure information 400, in which the three-dimensional-coordinate information for the α-helices, β-strands, and turns is synthesized, is outputted.

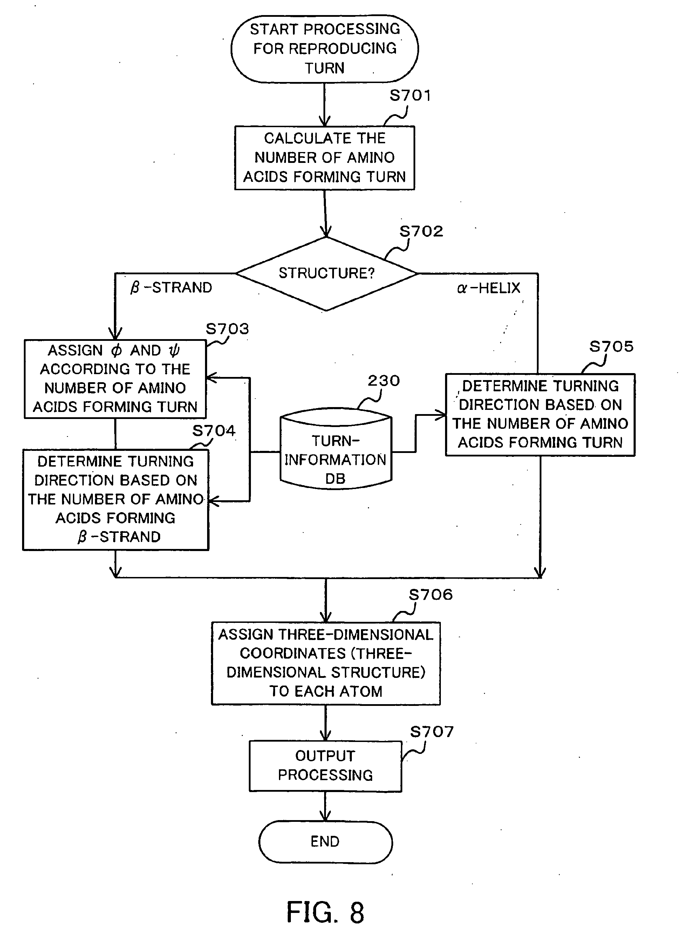

Hereinbelow, the processing for reproducing turns is explained. FIG. 8 is a flow diagram illustrating a sequence of processing for reproducing a turn.

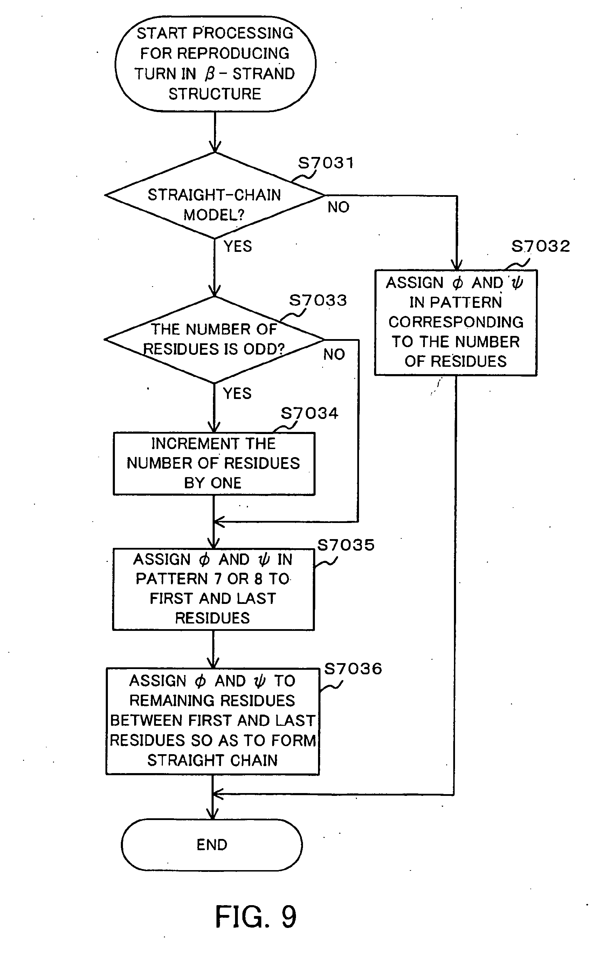

The processing for reproducing a turn is started after the secondary-structure information for a desired protein is generated or read in.
[Step S701] The number of amino acids at which a turn occurs is calculated based on the secondary-structure information. That is, the number of residues forming a turn is calculated.
[Step S702] Next, a structure in which the turn appears is checked. In the case where the turn is in a β-strand structure, the operation goes to step S703. In the case where the turn is in an α-helix structure, the operation goes to step S705. Although not shown, in the cases where the turn appears in another structure, the processing is branched in a similar manner.
[Step S703] In the case where the turn is in a β - strand structure, the turn-information DB 230 is searched for the angles φ and ψ based on the number of residues forming the turn, which is calculated in step S701, and values of the angles φ and ψ in a selected pattern are assigned. For example, since the number of residues forming a turn (68, 69) between a β-sheet (62-67) and a β-sheet (70-75) in the secondary-structure information 141 indicated in FIG. 4 is two, values of the angles φ and ψ prescribed for turns formed of two residues are searched. The numbers in the above parentheses indicate the positions of the amino acids in the secondary-structure information 141.
[Step S704] Next, the number of residues preceding the turn and forming a β-strand is calculated based on the secondary-structure information, and the turn-information DB 230 is searched based on the calculated number of the residues in order to determine the turning direction. For example, since, in the example mentioned in step S703, the number of residues in the β-sheet (62-67) is even, the turning direction is determined to be right. Alternatively, the turning direction may be determined by a program without the use of the turn-information DB 230, since the turning direction is determined to be left or right according to whether the number of residues forming each β-strand is odd or even, respectively. Thus, the dihedral angles and the turning direction in the β-strand structure are determined through steps S703 and S704, and the operation goes to step S706.
[Step S705] In the case where the turn is in an α-helix structure, the turn-information DB 230 is searched for the turning direction based on the number of residues forming the turn, which is calculated in step S701, and the turning direction is determined. For example, since the number of residues forming a turn (29-32) between an α-helix (2-28) and an α-helix (33-54) in the secondary-structure information 141 indicated in FIG. 4 is even, the α-helix (2-28) and the α-helix (33-54) are determined to be oriented in an identical direction. In addition, the corresponding angles φ and ψ are extracted from the turn-information DB 230. Alternatively, as in the case of the turn in a β-strand structure, the turning direction may be determined by a program without the use of the turn-information DB 230, since the turning direction is determined to orient the α-helices to opposite or identical directions according to whether the number of residues forming the turn is odd or even, respectively. Thus, the dihedral angles and the turning direction in the β-strand structure are determined, and the operation goes to step S706.
[Step S706] Based on the values of the angles and ψ and the turning directions which are determined in the above steps, the three-dimensional coordinates (representing the three-dimensional structure) are assigned to each atom, and the turn structures are assigned to three-dimensional coordinates.
[Step S707] Three-dimensional-coordinate information which is represented by three-dimensional coordinates and adapted to a predetermined output format is generated and outputted.
   Next, the processing in step S703 for assignment of the angles φ and ψ in the β-strand structure is explained in more detail. FIG. 9 is a flow diagram illustrating a sequence of processing for reproducing a turn in a β-strand structure. The processing of FIG. 9 is started when a detected turn is in a β-strand structure.
[Step S7031] It is determined whether or not prediction about the turn is to be made based on the straight-chain model. A condition for use of the straight-chain model, for example, a condition that the straight-chain model is used when the number of residues forming the turn exceeds five, is preset, and the above determination is made in accordance with the above condition. When it is determined that the straight-chain model is not to be used, the operation goes to step S7032. When it is determined that the straight-chain model is to be used, the operation goes to step S7033.
[Step S7032] When it is determined that the straight-chain model is not to be used, a pattern registered in the turn-information DB is selected according to the number of residues forming the turn, values of the angles φ and ψ in the pattern are assigned, and the processing is completed.
[Step S7033] When it is determined that the straight-chain model is to be used, it is determined whether or not the number of residues forming the turn is odd. When the number of residues forming the turn is odd, the operation goes to step S7034. When the number of residues forming the turn is even, step S7034 is skipped.
[Step S7034] When the number of residues forming the turn is odd, the number of residues is incremented by one. This is because residues forming the turn are arranged in a zigzag configuration, and no straight-chain structure can be realized when the number of residues forming the turn is odd.
[Step S7035] The values of the angles φ and ψ in the pattern 7 or 8 are assigned to the first and last ones of the residues forming the turn.
[Step S7036] Values of the angles φ and ψ are assigned to the remaining ones of the residues forming the turn between the first and last ones of the residues so that the remaining ones of the residues are arranged to form a straight chain.

According to the above processing, it is possible to predict structures of turns which are formed of any number of residues.

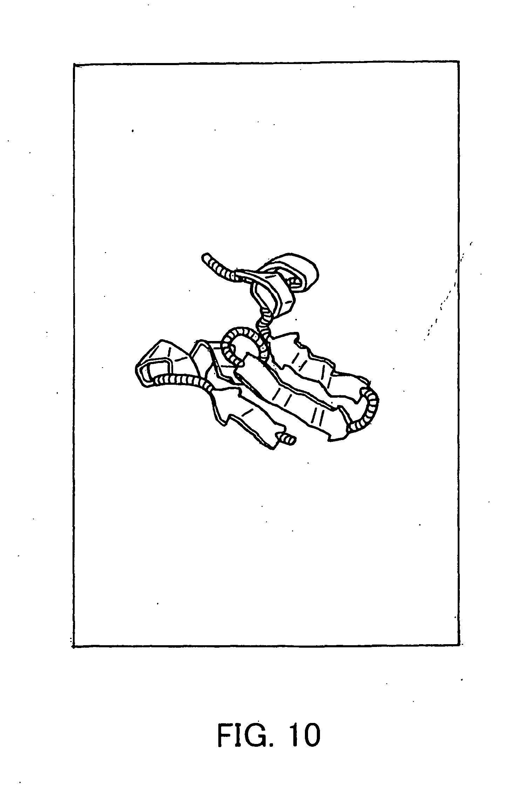

Based on the three-dimensional-coordinate information generated by the above procedure, the display unit 310 displays the three-dimensional structure of the protein. FIG. 10 is a diagram illustrating an example of display of a three-dimensional structure of a protein realized by the protein three-dimensional-structure prediction apparatus and the protein three-dimensional-structure prediction method according to an embodiment of the present invention. As in the drawings referred to before, in FIG. 10, β-strands are indicated by arrows, α-helices are indicated by helices, and portions connecting the β-strands and the α-helices are turns.

Since values of the angles φ and ψ are determined by referring to the turn-structure information, and probable turns are predicted, it is possible to reproduce turns which have high degrees of freedom and are difficult to determine by the conventional techniques. In the protein three-dimensional-structure prediction method according to the present invention, the structures of turns are predicted based on the secondary-structure information and the turn-structure information in which the structures of turns are registered in correspondence with the secondary structures. Therefore, it is possible to predict unknown three-dimensional structures of proteins each having an amino-acid sequence.

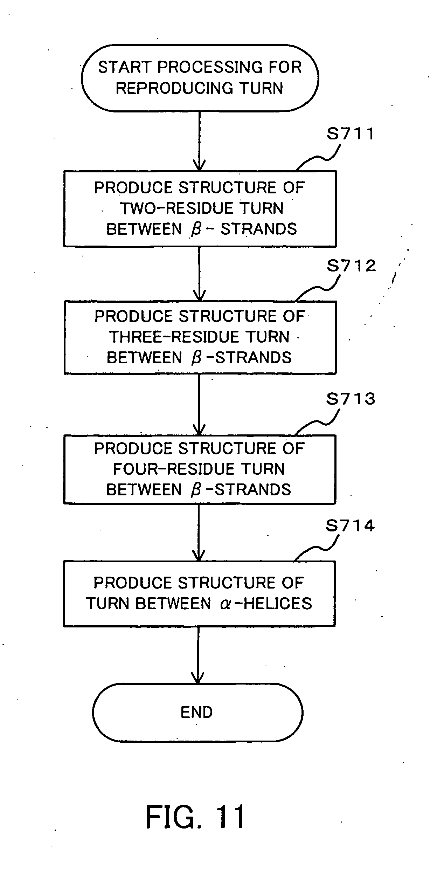

In the above explanations, every time a turn appears in an amino-acid sequence, the structure of the turn is analyzed. Alternatively, it is possible to reproduce turns on a type-by-type basis. FIG. 11 is a flow diagram illustrating a sequence of processing for reproducing a turn according to another embodiment of the present invention.

As in the processing of FIG. 8, the processing of FIG. 11 is started after secondary-structure information is generated.
[Step S711] From the secondary-structure information which has been read in, a portion of the secondary-structure information for at least one turn in a β-strand structure each of which is formed of two residues is extracted, the turn-information DB 230 is searched for values of the angles φ and ψ corresponding to the extracted portion of the secondary-structure information, and processing for determining a turning direction based on the number of residues forming a β-strand and preceding each turn for which the portion of the secondary-structure information is extracted is performed.
[Step S712] From the secondary-structure information which has been read in, a portion of the secondary-structure information for at least one turn in a β-strand structure each of which is formed of three residues is extracted, the turn-information DB 230 is searched for values of the angles φ and ψ corresponding to the extracted portion of the secondary-structure information, and processing for determining a turning direction based on the number of residues forming a β-strand and preceding each turn for which the portion of the secondary-structure information is extracted is performed.
[Step S713] From the secondary-structure information which has been read in, a portion of the secondary-structure information for at least one turn in a β-strand structure each of which is formed of four residues is extracted, the turn-information DB 230 is searched for values of the angles φ and ψ corresponding to the extracted portion of the secondary-structure information, and processing for determining a turning direction based on the number of residues forming a β-strand and preceding each turn for which the portion of the secondary-structure information is extracted is performed.
[Step S714] From the secondary-structure information which has been read in, a portion of the secondary-structure information for at least one turn in an α-helix structure is extracted, the turn-information DB 230 is searched for values of the angles φ and ψ corresponding to the extracted portion of the secondary-structure information, and processing for determining a turning direction based on the number of residues forming each turn for which the portion of the secondary-structure information is extracted is performed.

Thus, the structures of turns can be predicted by the above procedure. Although the processing using the straight-chain model is not included in the above sequence in order to simplify the explanations, it is possible to add such processing to the above sequence, for example, by inserting between steps S713 and S714 processing using the straight-chain model as processing to be performed in the cases where the number of residues is five or more.

In addition, it is possible to obtain the turn-structure information registered in the turn-information DB 230 in the above explanations by performing three-dimensional structural analysis of proteins having known three-dimensional structures. In particular, the present inventor has succeeded in finding, by three-dimensional structural analysis, rules for determining dihedral angles and turning directions of turns appearing between β-strands in β-strand structures and turning directions of turns appearing between α-helices in α- helix structures.

Hereinbelow, the three-dimensional structural analysis of proteins having known three-dimensional structures performed by the present inventor is explained. First, analysis of turns in β-strand structures is explained, and then analysis of turns in α-helix structures is explained.

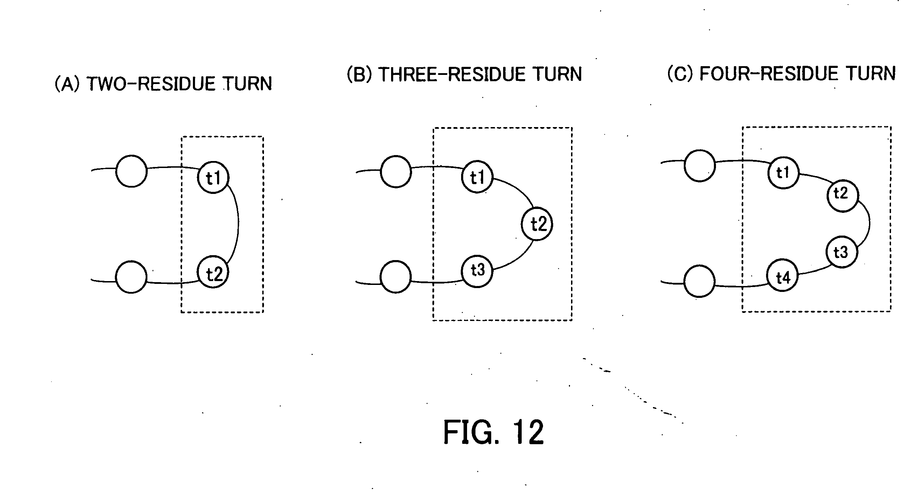

For example, when a β-sheet is formed from a β-strand structure, the β- strand structure is folded by 180-degree turns. In this case, at least two residues are necessary for each 180-degree turn. FIG. 12 is a diagram illustrating classification into different types of turns used in three-dimensional structural analysis of proteins. A turn realized by two residues is illustrated in the part (A) of FIG. 12, a turn realized by three residues is illustrated in the part (B) of FIG. 12, and a turn realized by four residues is illustrated in the part (C) of FIG. 12. Each residue constituting a turn is indicated by tn, where n = 1, 2, .... FIG. 12 is provided only for the purpose of illustrating classification of turns, and does not show actual structures. Since most turns are formed of two to four residues, it is possible to predict the structures of most turns in β-strand structures by performing three-dimensional structural analysis of turns each of which is realized by two, three, and four residues, and deriving probable values of the angles φ and ψ. In addition, as explained before, turns formed of three or more residues can be predicted by using the straight-chain model. In this case, the first and last ones of the residues forming each turn are respectively realized by the residues t1 and t2 in the turns of the type illustrated in the part (A) of FIG. 12, and the remaining ones of the residues forming the turn are arranged in the form of a straight chain between the first and last ones of the residues forming the turn.

Next, the three-dimensional structural analysis which the present inventor has performed in each of the above cases is explained below.

In order to perform analysis of three-dimensional structures, first, the present inventor has searched the PDB (Protein Data Bank) for β-strand-rich proteins by using a keyword "porin," where the PDB is a data bank of three-dimensional structures of proteins which is managed by Research Collaboratory for Structural Bioinfomatics (RCSB) in the United States, and the β-strand-rich proteins are proteins which are rich in β-strand structures. The present inventor has accessed the PDB site through the Internet and other networks, and searched for the desired data. Thus, 138 proteins have been obtained by the search.

Subsequently, analysis of turns of respective types is performed. As understood from FIG. 12, in the case of the type illustrated in the part (A) of FIG. 12, a 180-degree turn is realized by two residues. Hereinafter, turns of this type are referred to as 180-degree β-strand turns. In the case of the type illustrated in the part (B) of FIG. 12, a 180-degree turn is realized by three residues (i.e., a 90-degree turn is realized by each residue). Hereinafter, turns of this type are referred to as 90-degree β-strand turns. In the case of the type illustrated in the part (C) of FIG. 12, a 180-degree turn is realized by four residues (i.e., a 60-degree turn is realized by each residue). Hereinafter, turns of this type are referred to as 60-degree β -strand turns.

First, analysis of 180-degree turns of the type (A) each of which is realized by two residues (i.e., 180-degree β-strand turns) is explained below. The present inventor has searched the proteins obtained by the aforementioned search of the PDB, for portions in which actually 180-degree turns are each realized by two residues. In the search for the 180-degree β-strand turns, the structures are investigated, for example, by using Protein Adviser for Win (FQS), and the angles and ψ are investigated by using the free software, DSSP. Thus, the present inventor has found fifty-nine 180-degree turns each of which is realized by two residues. Subsequently, the present inventor has produced Ramachandran plots based on the values of the angles φ and ψ in the fifty-nine 180-degree turns. In the Ramachandran plots, data of the angles φ and ψ in the respective amino acids are plotted on planes each having an abscissa corresponding to the angle φ and an ordinate corresponding to the angle ψ. In actual proteins, the allowable ranges of the dihedral angles are limited by steric hindrance. When a Ramachandran plot is produced, it is possible to recognize the allowable ranges.

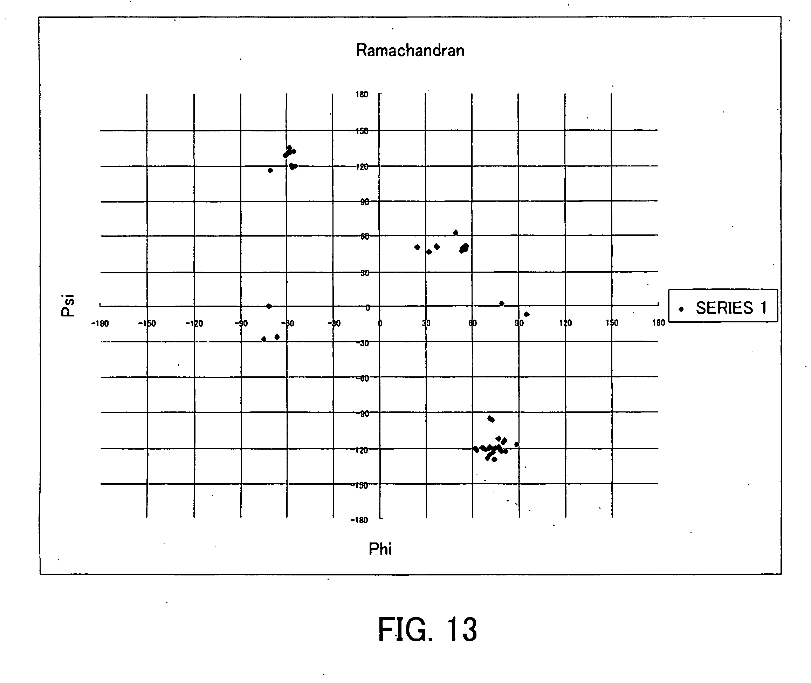

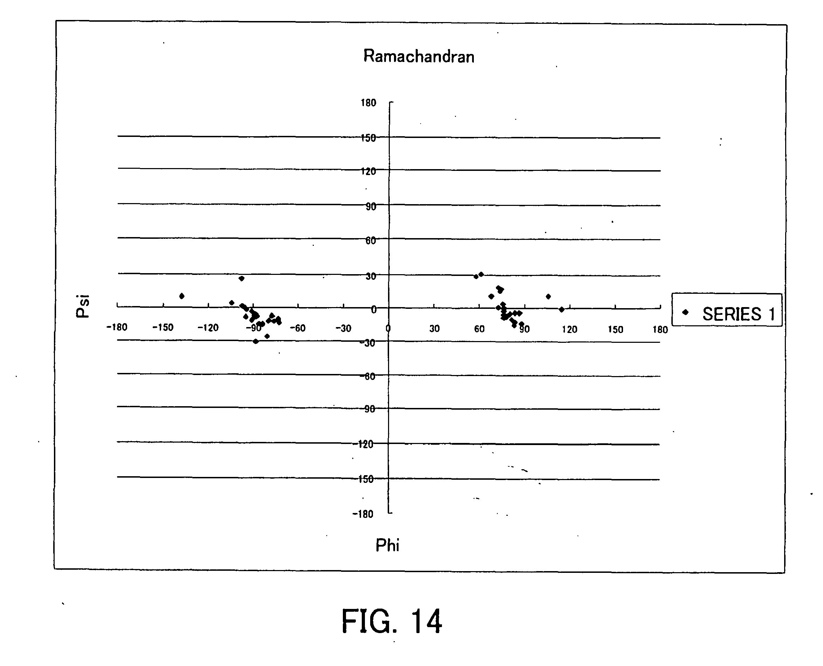

FIG. 13 is a Ramanchandran plot of the first residues in the 180-degree β-strand turns, and FIG. 14 is a Ramanchandran plot of the second residues in the 180-degree β-strand turns. In each of the Ramachandran plots, data of the angles φ and ψ in the amino acid as each of the first and second residues in the fifty-nine 180-degree β-strand turns are plotted on a plane having an abscissa corresponding to the angle φ and an ordinate corresponding to the angle ψ. FIGS. 13 and 14 show that data are concentrated in specific areas. That is, the combinations of the angles φ and ψ in most of the actual three-dimensional structures are in the areas.

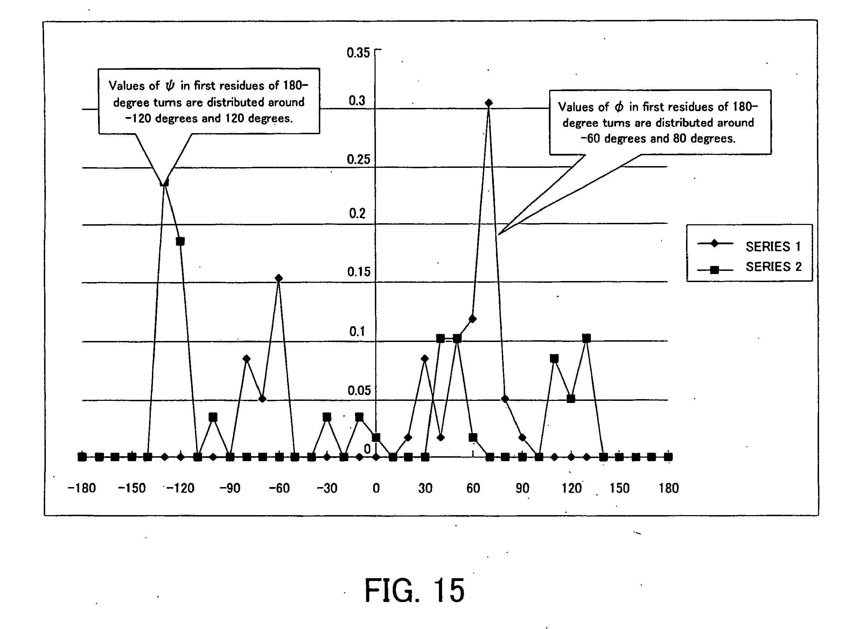

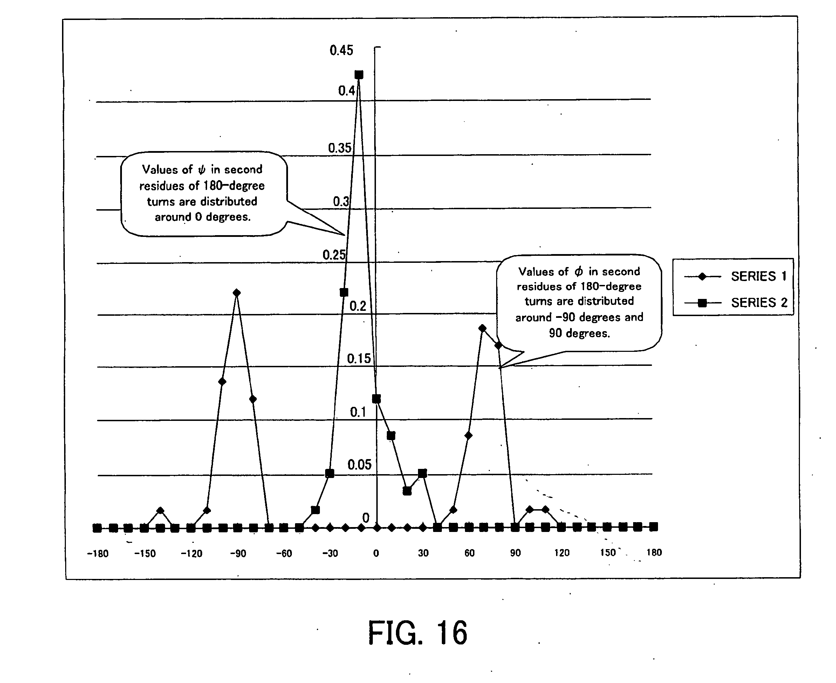

In order to clarify the distributions of the angles φ and ψ, angular distributions of the first and second residues are indicated. FIG. 15 is a graph indicating distributions of the angles φ and ψ in the first residues in 180-degree β-strand turns. In the graph of FIG. 15, the range of each of the angles φ and ψ from -180 degrees to 180 degrees is divided into sections each having a width of 10 degrees, and the probability that each of the angles φ and ψ is in each section is indicated, where the probability is normalized so that the sum of the probabilities in the entire range is one. According to the distribution of FIG. 15, the values of the angle φ in the first residues of the 180-degree β-strand turns are mainly distributed around -60 degrees and 80 degrees, and the values of the angle ψ in the first residues of the 180-degree β - strand turns are mainly distributed around -120 degrees and 120 degrees. In addition, FIG. 16 is a graph indicating distributions of the angles φ and ψ in the second residues in the 180-degree β-strand turns. According to the distribution of FIG. 16, the values of the angle φ in the second residues of the 180-degree β-strand turns are mainly distributed around -90 degrees and 90 degrees, and the values of the angles ψ in the second residues of the 180-degree β-strand turns are mainly distributed around 0 degrees. Thus, probable values of the angles φ and ψ in the 180-degree β -strand turns are obtained from the distributions.

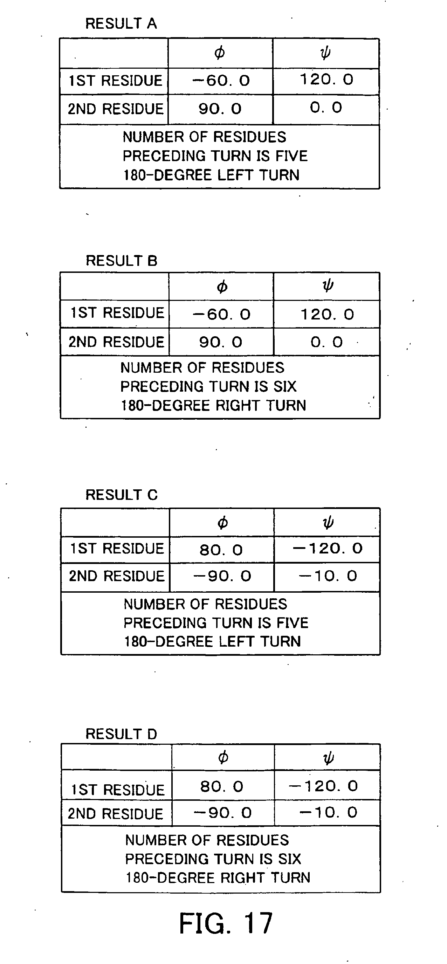

FIG. 17 shows representative values of the dihedral angles φ and ψ in and corresponding turning directions of 180-degree β-strand turns, which are obtained by three-dimensional structural analysis.

In FIG. 17, the result A indicates that the angle φ is -60.0 degrees and the angle ψ is 120.0 degrees in the first residue of each turn, the angle φ is 90.0 degrees and the angle ψ is 0.0 degrees in the second residue of the turn, the number of residues forming a strand and preceding the turn is five, and the turning direction is left. FIGS. 15 and 16 show that the combinations of the above angles φ and ψ are probable. The result B indicates that the combination of the angles φ and ψ in the result B is identical to the combination of the angles φ and ψ in the result A, the number of residues forming a β-strand and preceding each turn in the result B is six, and the turning direction is right. The result C indicates that the angle φ is around 80.0 degrees and the angles ψ is around -120.0 degrees in the first residue of each turn, the angle φ is around -90.0 degrees and the angles ψ is around -10.0 degrees in the second residue of the turn, the number of residues forming a β-strand and preceding the turn is five, and the turning direction is left. The result D indicates that the combination of the angles and ψ in the result D is identical to the combination of the angles φ and ψ in the result C, the number of residues forming a β-strand and preceding each turn in the result D is six, and the turning direction is right.

Further, the present inventor has investigated the results of the three-dimensional structural analysis indicated in FIG. 17, and found that each turn between β-strands can be classified into a left turn or a right turn according to whether the number of residues forming a β-strand and preceding the turn is odd or even, respectively.

The patterns of the angles φ and ψ determined as above are stored in the turn-information DB 230 in advance. In order to reproduce a turn, the turn prediction means 220 searches the turn-information DB 230, and reads out and utilizes the patterns.

Next, examples of three-dimensional structures having probable dihedral angles obtained as above, which are displayed by the protein three-dimensional-structure prediction apparatus according to the present invention, are explained below.

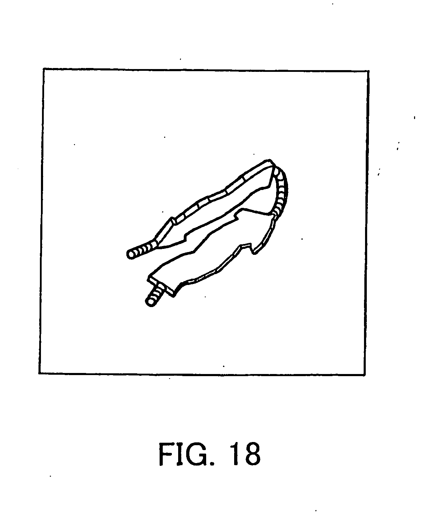

FIG. 18 shows an example of display of a first three-dimensional structure of a 180-degree β-strand turn, where the first three-dimensional structure is determined by the data of the dihedral angles and the number of residues which are indicated in the result C in FIG. 17. In the example of FIG. 18, the portions illustrated in the form of an arrow are β-strands and the portion connecting the β-strands is a turn. The turn illustrated in FIG. 18 is an example of a left turn.

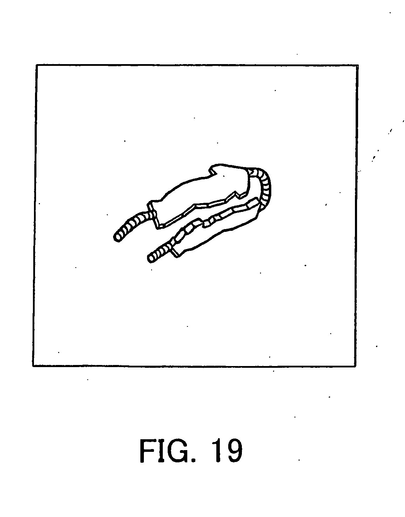

Similarly, FIG. 19 shows an example of display of a second three-dimensional structure of a 180-degree β-strand turn, where the second three-dimensional structure is determined by the data of the dihedral angles and the number of residues which are indicated in the result D in FIG. 17. Although the angles φ and ψ in the example of FIG. 19 are identical to the angles and ψ in the example of FIG. 18, a right turn occurs in FIG. 19.

Before registration as turn-structure information in a database, the angles φ and ψ in each turn obtained from the graphs of distributions are reproduced by using modeling software in which the protein three-dimensional-structure prediction method according to the present invention is used, and the turn is visually confirmed by using display software. Then, the angles in the turn are finely tuned, and angles to be registered are determined.

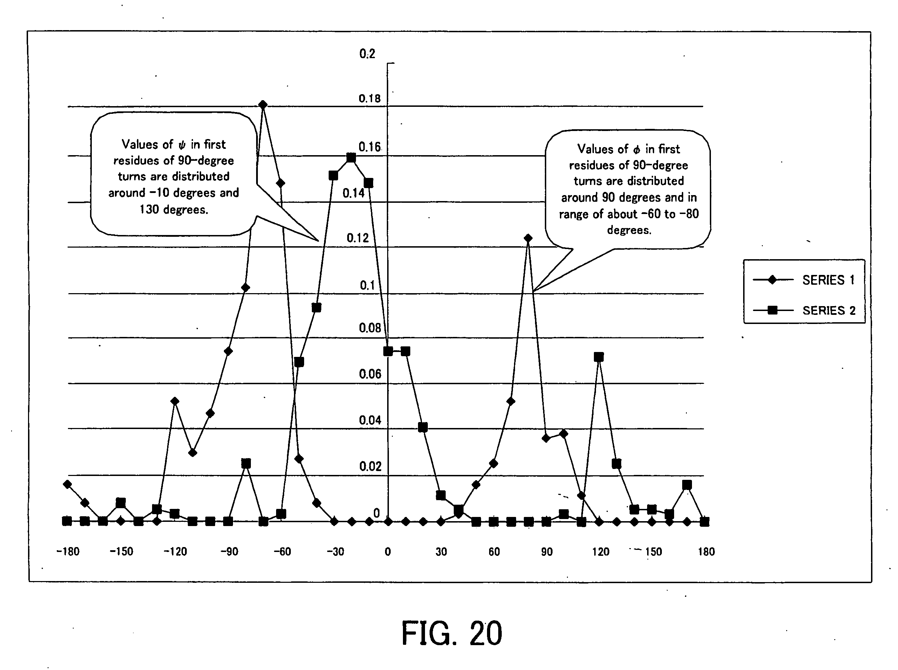

Next, analysis of 90-degree turns of the type (B) each of which is realized by three residues (i.e., 90-degree β-strand turns) is explained below. The aforementioned 138 proteins obtained by the search of the PDB are further searched for portions in each of which a 90-degree turn is actually realized by a residue. In a similar manner to the case of the 180-degree β-strand turn of the type (A), the present inventor has found three hundred and sixty-five 90-degree β-strand turns. Subsequently, Ramachandran plots are produced based on the values of the angles φ and ψ in the three hundred and sixty-five 90-degree turns. Further, in order to clarify the distributions of the angles φ and ψ, angular distributions are indicated. FIG. 20 is a graph indicating distributions of the angles φ and ψ in the first residues in the 90-degree turns. The distributions in FIG. 20 are plotted in a similar manner to the case of the 180-degree β-strand turn. According to the distribution of FIG. 20, the values of the angle φ in the first residues of the 90-degree β-strand turns are mainly distributed around 90 degrees and in a range of about -60 degrees to -80 degrees, and the values of the angle ψ in the first residues of the 90-degree β-strand turns are mainly distributed around -10 degrees and 130 degrees. Thus, probable values of the angles φ and ψ in the 90-degree β-strand turns are obtained.

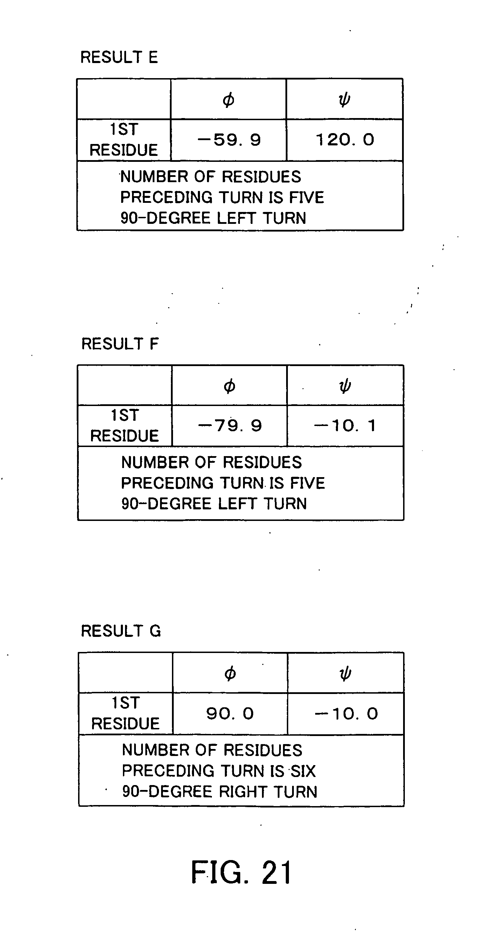

FIG. 21 shows representative values of the dihedral angles φ and ψ in and corresponding turning directions of 90-degree β-strand turns, which are obtained by three-dimensional structural analysis.

In FIG. 21, the result E indicates that the angle φ is -59.9 degrees and the angle ψ is 120.0 degrees in the first residue of each turn, the number of residues forming a β-strand and preceding the turn is five, and the turning direction is left. The result F indicates that the angle φ is around -79.9 degrees and the angle ψ is around -10.1 degrees in the first residue of each turn, the number of residues forming a β-strand and preceding the turn is an odd number, five, and the turning direction is left. The result G indicates that the angle φ is around 90.0 degrees and the angle ψ is around -10.0 degrees in the first residue of each turn, the number of residues forming a -strand and preceding the turn is six, and the turning direction is right.

Similar to the case of the 180-degree β-strand turn, examples of three-dimensional structures having probable dihedral angles obtained as above, which are displayed by the protein three-dimensional-structure prediction apparatus according to the present invention, are explained below.

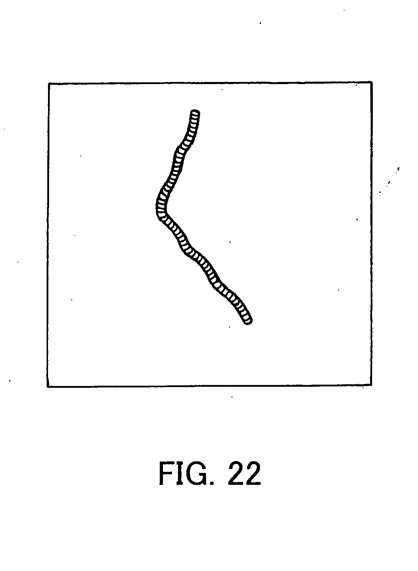

FIG. 22 shows an example of display of a first three-dimensional structure of a 90-degree β-strand turn. Specifically, FIG. 22 shows an example of a display screen in which a turning portion in a left turn determined by the data of the dihedral angles in the result E in FIG. 21 is indicated.

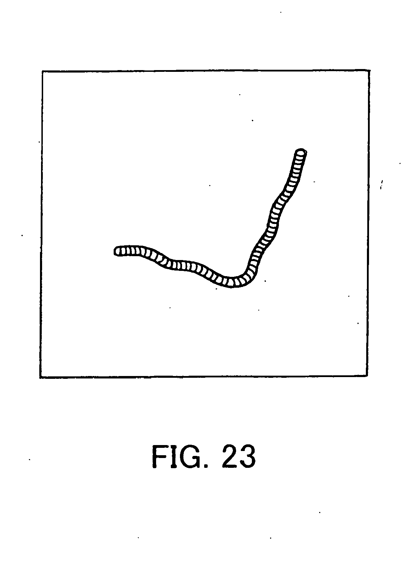

Similarly, FIG. 23 shows an example of display of a second three-dimensional structure of a 90-degree β-strand turn. Specifically, FIG. 23 shows an example of a display screen in which a turning portion in a right turn determined by the data of the dihedral angles in the result G in FIG. 21 is indicated.

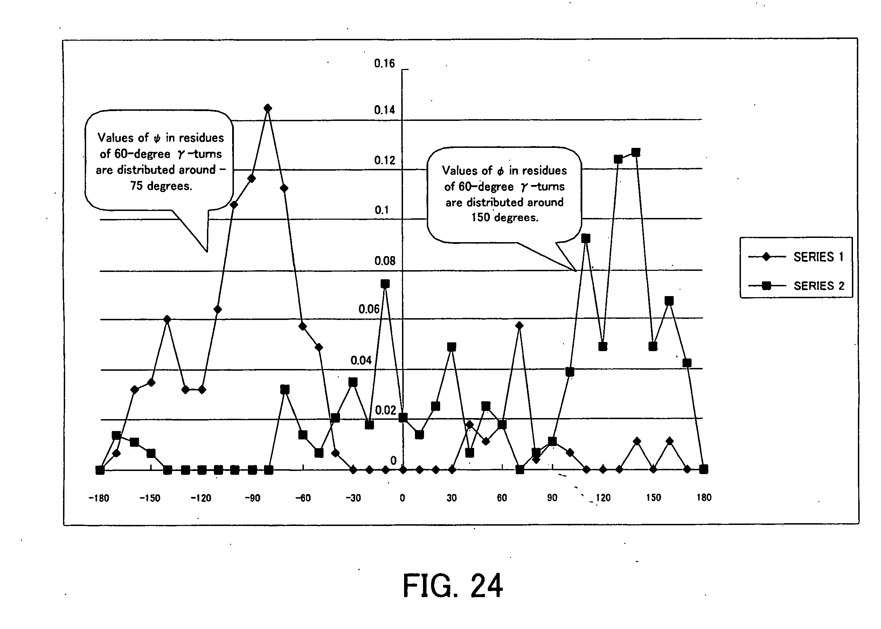

Next, analysis of 60-degree turns of the type (C) each of which is realized by four residues (i.e., 60-degree β-strand turns) is explained below. The aforementioned 138 proteins obtained by the search of the PDB are further searched for portions in each of which a 60-degree turn is actually realized by a residue. In a similar manner to the case of the 180-degree β-strand turn of the type (A), the present inventor has found two hundred and seventy-three 60-degree β-strand turns. Subsequently, Ramachandran plots are produced based on the values of the angles φ and ψ in the two hundred and seventy-three 60-degree turns. Further, in order to clarify the distributions of the angles φ and ψ, angular distributions are indicated. FIG. 24 is a graph indicating distributions of the angles φ and ψ in the first residues in the 60-degree β-strand turns. The distributions in FIG. 24 are plotted in a similar manner to the case of the 180-degree β-strand turn of the type (A). According to the distribution of FIG. 24, the values of the angle φ in the first residues of the 60-degree β-strand turns are mainly distributed around 150 degrees, and the values of the angle ψ in the first residues of the 60-degree β-strand turns are mainly distributed around -75 degrees. Thus, probable values of the angles φ and ψ in the 60-degree β-strand turns are obtained.

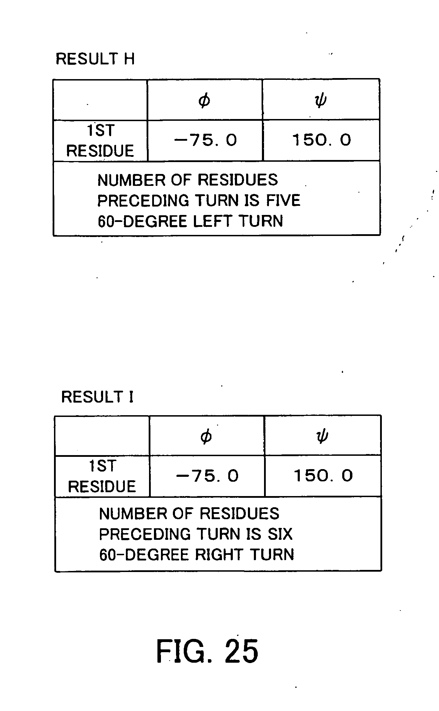

FIG. 25 shows probable values of the dihedral angles φ and ψ in 60-degree β-strand turns, which are obtained by three-dimensional structural analysis.

In FIG. 25, the result H indicates that the angle φ is -75.0 degrees and the angle ψ is 150.0 degrees in the first residue of each turn, the number of residues forming a β-strand and preceding the turn is an odd number, five, and the turning direction is left. The result I indicates that the combination of the angles φ and ψ in the result I is identical to the combination of the angles φ and ψ in the result H, the number of residues forming a β-strand and preceding the turn in the result I is six, and the turning direction is right.

Thereafter, examples of three-dimensional structures having probable dihedral angles obtained as above, which are displayed by the protein three-dimensional-structure prediction apparatus according to the present invention, are explained below.

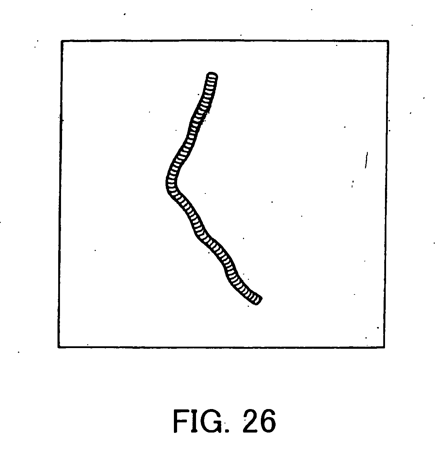

FIG. 26 is a diagram illustrating an example of display of a first three-dimensional structure of a 60-degree β-strand turn. Specifically, FIG. 26 shows an example of a display screen in which a turning portion in a left turn determined by the data of the dihedral angles in the result H in FIG. 25 is indicated.

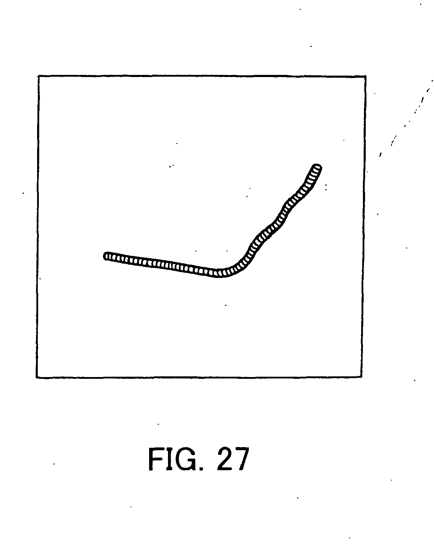

Similarly, FIG. 27 is a diagram illustrating an example of display of a second three-dimensional structure of a 60-degree β-strand turn. Specifically, FIG. 27 shows an example of a display screen in which a turning portion in a right turn determined by the data of the dihedral angles in the result I in FIG. 25 is indicated.

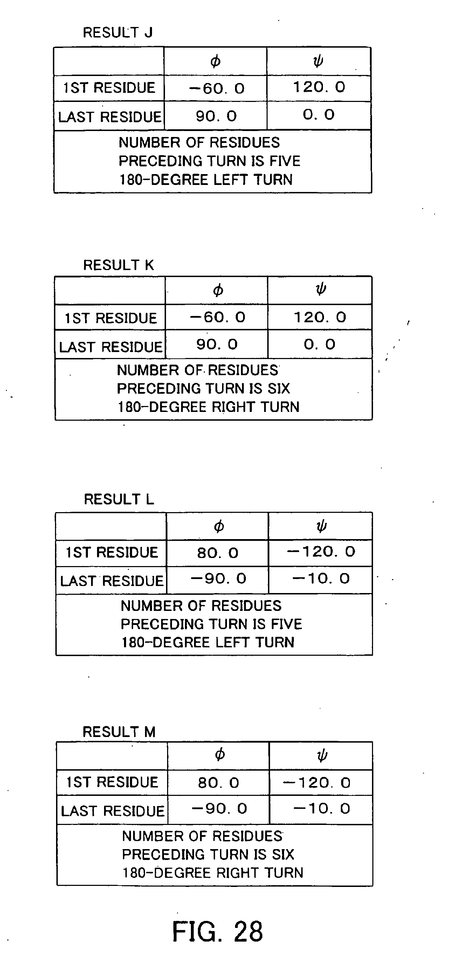

Next, analysis based on the straight-chain model is explained below. The present inventor has performed three-dimensional structural analysis based on the straight-chain model in a similar manner to the analysis of the structures of the turns in the β-strand structures explained above. In the case of the straight-chain model, similar to the case of the 180-degree turn of the type (A) formed of the two residues t1 and t2, each turn is realized by the first and last residues (respectively corresponding to the residues t1 and t2 in the 180-degree turn), and the remaining residues between the first and last residues are arranged to form a straight line. FIG. 28 shows probable values of the dihedral angles φ and ψ in structures obtained by three-dimensional structural analysis in accordance with the straight-chain model.

In FIG. 28, the result J indicates that the angle φ is -60.0 degrees and the angle ψ is 120.0 degrees in the first residue of each turn, the angle φ is 90.0 degrees and the angle ψ is 0.0 degrees in the last residue of the turn, the number of residues forming a β-strand and preceding the turn is five, and the turning direction is left. The result K indicates that the combination of the angles φ and ψ in the result K is identical to the combination of the angles φ and ψ in the result J, the number of residues forming a β-strand and preceding each turn in the result K is six, and the turning direction is right. The result L indicates that the angle φ is 80.0 degrees and the angle ψ is -120.0 degrees in the first residue of each turn, the angle φ is -90.0 degrees and the angle ψ is -10.0 degrees in the second residue of the turn, the number of residues forming a β-strand and preceding the turn is five, and the turning direction is left. The result M indicates that the combination of the angles φ and ψ in the result M is identical to the combination of the angles φ and ψ in the result L, the number of residues forming a β-strand and preceding the turn in the result M is six, and the turning direction is right.

Next, analysis of 180-degree turns in α-helix structures (180-degree α-helix turns) is explained below. The present inventor has also performed three-dimensional structural analysis of 180-degree turns in α-helix structures in a similar manner to the analysis of the structures of the turns in the β-strand structures explained before, and found the following features which depend on the number of residues arranged between first and second α-helices when each α-helix structure contains the first and second α-helices and is bent at the residues arranged between the first and second α - helices:
When the number of the residues is odd, the first and second α-helices are oriented in opposite directions (i.e., a 180-degree turn occurs).
When the number of the residues is even, the first and second α-helices are oriented in an identical direction.

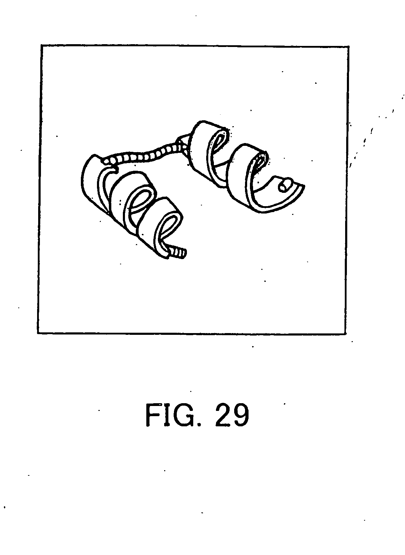

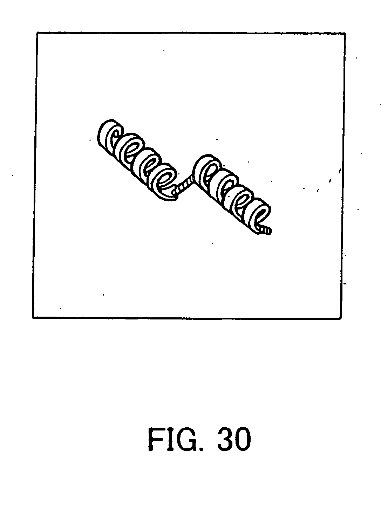

Although the number of residues forming a turn in actual α-helix structures can be any number, it is possible to predict and reproduce structures of turns in α-helix structures based on the above features. Examples of display of turns in α-helix structures by the protein three-dimensional-structure prediction apparatus according to the present invention in accordance with the above rules are indicated below. FIG. 29 is a diagram illustrating an example of display of a first three-dimensional structure of a turn in an α-helix structure. In FIG. 29, α-helices and a turn in an α-helix structure in which the number of residues forming the turn is odd are illustrated. Since the number of residues forming the turn is odd, a 180-degree turn is realized. FIG. 30 is a diagram illustrating an example of display of a second three-dimensional structure of a turn in an α-helix structure. In FIG. 30, α-helices and a turn in an α-helix structure in which the number of residues forming the turn is even are illustrated. Since the number of residues forming the turn is even, the turn is formed so that the α-helices are oriented in an identical direction.

In the above explanations, only examples of patterns in the turn-structure information which is registered in the turn-information DB 230 are illustrated. However, the present invention is not limited to such examples. The patterns in the turn-structure information registered in the turn-information DB 230 are arbitrary. It is possible to register a plurality of patterns so that an arbitrary one of the patterns can be chosen. Alternatively, it is possible to register only a pattern which is chosen in advance for use.

The above processing functions can be realized by a computer. In this case, a program describing details of processing for realizing the functions which the protein three-dimensional-structure prediction apparatus should have is provided. When the computer executes the program, the above processing functions can be realized on the computer.

The program describing the details of the processing can be stored in a recording medium which can be read by the computer. The recording medium may be a magnetic recording device, an optical disk, an optical magnetic recording medium, a semiconductor memory, or the like. The magnetic recording device may be a hard disk drive (HDD), a flexible disk (FD), a magnetic tape, or the like. The optical disk may be a DVD (Digital Versatile Disk), a DVD-RAM (Random Access Memory), a CD-ROM (Compact Disk Read Only Memory), a CD-R (Recordable)/RW (ReWritable), or the like. The optical magnetic recording medium may be an MO (Magneto-Optical Disk) or the like.

In order to put the program into the market, for example, it is possible to sell a portable recording medium such as a DVD or a CD-ROM in which the program is recorded. Alternatively, it is possible to store the program in a storage device belonging to a server computer, and transfer the program to another computer through a network.

The computer which executes the program stores the program in a storage device belonging to the computer, where the program is originally recorded in, for example, a portable recording medium. The computer reads the program from the storage device, and performs processing in accordance with the program. Alternatively, the computer may directly read the program from the portable recording medium for performing processing in accordance with the program. Further, the computer can sequentially execute processing in accordance with each portion of the program every time the portion of the program is transferred from the server computer.

As explained above, the protein three-dimensional-structure prediction apparatus according to the present invention reads in an amino-acid sequence as a primary sequence of a protein, predicts secondary structures or acquires secondary structures from a database, calculates the number of amino acids forming a turn based on the predicted secondary structures, acquires a probable turn structure which is obtained in correspondence with the secondary structures and the number of the amino acids, reproduces the turn, and predicts a three-dimensional structure.

Since the turn is predicted based on the probable turn structure which is obtained in correspondence with the secondary structures and the number of the amino acids forming the turn, it is possible to reproduce the turn which has a high degree of freedom and cannot be determined by the conventional techniques. Therefore, even when the structure of a protein corresponding to an amino-acid sequence is unknown, the structure of the protein can be predicted.

In addition, when a computer executes the protein three-dimensional-structure prediction program according to the present invention, the computer reads in an amino-acid sequence of a protein, and acquires secondary-structure information. Next, the computer calculates the number of amino acids forming a turn, based on the secondary-structure information, predicts and reproduces the turn by acquiring turn-structure information on a probable turn structure based on the secondary-structure information and the calculated number of the amino acids, and predicts a three-dimensional structure of the protein.

As described above, turn-structure information on a probable turn structure is acquired based on secondary structures and the number of amino acids forming a turn, and the turn is predicted, where the number of the amino acids is obtained from the secondary structures. Therefore, it is possible to predict and reproduce the turn which has a high degree of freedom and cannot be determined by the conventional techniques. Thus, even when the structure of a protein corresponding to an amino-acid sequence is unknown, the structure of the protein can be predicted.

## Claims

1. A protein three-dimensional-structure prediction apparatus for predicting based on a sequence of amino acids a three-dimensional structure of a protein formed of the amino acids, **characterized in** comprising:
a turn-forming-portion calculation means which calculates the number of amino acids forming a turn, based on said sequence of the amino acids and local secondary-structure information of a secondary-structure, where the sequence of the amino acids is read in as a primary sequence of said protein, and the local secondary-structure information is obtained from the sequence of the amino acids;
a turn prediction means which acquires turn-structure information on a probable turn, according to said local secondary-structure information and said number of the amino acids forming said turn, and reproduces a turning portion of the three-dimensional structure of said protein based on the turn-structure information, where the turn-structure information is extracted from three-dimensional-structure information for proteins having known three-dimensional structures; and
a three-dimensional-structure reproduction means which generates reproduction information in which the three-dimensional structure of said protein is reproduced by using the reproduced turning portion.

2. The protein three-dimensional-structure prediction apparatus according to claim 1, wherein said turn prediction means comprises,
a turn-information storage means which stores said turn-structure information in association with said secondary-structure and said number of the amino acids forming said turn, where the turn-structure information is extracted in advance from said three-dimensional-structure information according to the local secondary-structure information and the number of the amino acids forming the turn, and
a search means which acquires said turn-structure information by searching said turn-information storage means based on said secondary-structure and the number of the amino acids forming said turn.

3. The protein three-dimensional-structure prediction apparatus according to claim 2, wherein probable dihedral angles which are formed by peptide planes and extracted in advance from said three-dimensional-structure information are stored in said turn-information storage means in correspondence with secondary structures between which said turn appears and said number of the amino acids forming the turn, and
said search means comprises a dihedral-angle assignment means which searches said turn-information storage means for said dihedral angles corresponding to said secondary-structures and said number of the amino acids forming the turn, and performs assignment of said dihedral angles.

4. The protein three-dimensional-structure prediction apparatus according to claim 3, wherein said turn-information storage means stores, in correspondence with said number of the amino acids forming the turn, dihedral angles which are probable when said turn appears between β-strands and is a 180-degree β-strand turn realized by a turn of a predetermined angle occurring at each of said amino acids forming the turn.

5. The protein three-dimensional-structure prediction apparatus according to claim 3, wherein said turn-information storage means stores, in correspondence with said number of the amino acids forming the turn, dihedral angles which are probable when said turn appears between β-strands, and the first and last ones of said amino acids forming the turn is a 180-degree β-strand turn realized in such a manner that other ones of said amino acids forming the turn which are located between the first and last ones are bonded to form a straight chain.

6. The protein three-dimensional-structure prediction apparatus according to claim 1, wherein said turn prediction means comprises a turning-direction calculation means which determines the direction of the turn according to said secondary-structure and, when necessary, said number of the amino acids forming the turn.

7. The protein three-dimensional-structure prediction apparatus according to claim 6, wherein when said turn appears between β-strands, said turning-direction calculation means determines the direction of the turn according to whether the number of the amino acids forming a β -strand and preceding said turn is odd or even.

8. The protein three-dimensional-structure prediction apparatus according to claim 6, wherein when said turn appears between α-helices, said turning-direction calculation means determines the direction of the turn according to whether the number of the amino acids forming said turn is odd or even.

9. A computer-implemented protein three-dimensional-structure prediction method for predicting based on a sequence of amino acids a three-dimensional structure of a protein formed of the amino acids, **characterized in** comprising:
a step of acquiring in advance and storing in a predetermined storage means turn-structure information on probable turns corresponding to local secondary-structure information and the number of amino acids forming each of the probable turns, where the turn-structure information is extracted from three-dimensional-structure information for proteins having known three-dimensional structures;
a step of acquiring said sequence of the amino acids as a primary sequence of said protein and said local secondary-structure information which is obtained from the sequence of the amino acids;
a step of calculating the number of the amino acids forming a turn, based on said local secondary-structure information;
a step of extracting a portion of said turn-structure information corresponding to said local secondary-structure information and said number of the amino acids forming said turn, by searching said predetermined storage means for the portion of the turn-structure information based on the local secondary-structure information and the number of the amino acids forming said turn; and
a step of generating reproduction information in which the three-dimensional structure of said protein is reproduced by using a turning portion reproduced based on the extracted portion of the turn-structure information.

10. The protein three-dimensional-structure prediction method according to claim 9, wherein in said step of extracting said turn-structure information, turning portions in said protein the three-dimensional structure of which is to be predicted are searched based on said local secondary-structure information, and a portion of the turn-structure information corresponding to said turn is extracted when said turn is detected.

11. The protein three-dimensional-structure prediction method according to claim 9, wherein in said step of extracting said turn-structure information, said protein the three-dimensional structure of which is to be predicted is searched, based on said local secondary-structure information, for said turning portion to which said turn-structure information extracted according to said secondary-structure information and the number of the amino acids forming the turn is applied.

12. A program for predicting based on a sequence of amino acids a three-dimensional structure of a protein formed of the amino acids, by using a computer, **characterized in that** said program makes said computer comprise functions of:
a turn-forming-portion calculation means which calculates the number of amino acids forming a turn, based on said sequence of the amino acids and local secondary-structure information, where the sequence of the amino acids is read in as a primary sequence of said protein, and the local secondary-structure information is obtained from the sequence of the amino acids;
a turn prediction means which acquires turn-structure information on a probable turn, according to said local secondary-structure information and said number of the amino acids forming said turn, and reproduces a turning portion of the three-dimensional structure of said protein based on the turn-structure information, where the turn-structure information is extracted from three-dimensional-structure information for proteins having known three-dimensional structures; and
a three-dimensional-structure reproduction means which generates reproduction information in which the three-dimensional structure of said protein is reproduced by using the reproduced turning portion.

13. A computer-readable recording medium storing a program for predicting based on a sequence of amino acids a three-dimensional structure of a protein formed of the amino acids, by using a computer, **characterized in that** said program makes said computer comprise functions of:
a turn-forming-portion calculation means which calculates the number of amino acids forming a turn, based on said sequence of the amino acids and local secondary-structure information, where the sequence of the amino acids is read in as a primary sequence of said protein, and the local secondary-structure information is obtained from the sequence of the amino acids;
a turn prediction means which acquires turn-structure information on a probable turn, according to said local secondary-structure information and said number of the amino acids forming said turn, and reproduces a turning portion of the three-dimensional structure of said protein based on the turn-structure information, where the turn-structure information is extracted from three-dimensional-structure information for proteins having known three-dimensional structures; and
a three-dimensional-structure reproduction means which generates reproduction information in which the three-dimensional structure of said protein is reproduced by using the reproduced turning portion.

## Patentansprüche

1. Eine Vorhersagevorrichtung für eine dreidimensionale Struktur eines Proteins zum Vorhersagen einer dreidimensionalen Struktur eines Proteins auf Grundlage einer Aminosäuresequenz, wobei das Protein aus den Aminosäuren ausgebildet ist, die **dadurch gekennzeichnet ist, dass** sie umfasst:
ein Mittel zum Berechnen eines Windungs-Ausbildungs-Abschnitts, das die Anzahl an eine Windung ausbildenden Aminosäuren auf Grundlage der Aminosäuresequenz und lokaler Sekundärstruktur-Information einer Sekundärstruktur berechnet, wobei die Aminosäuresequenz als eine Primärsequenz des Proteins eingelesen wird, und die lokale Sekundärstruktur-Information aus der Aminosäuresequenz erhalten wird;
ein Mittel zum Vorhersagen einer Windung, das Windungsstruktur-Information über eine wahrscheinliche Windung gemäß der lokalen Sekundärstruktur-Information und der Anzahl der die Windung ausbildenden Aminosäuren gewinnt, und das einen Windungs-Abschnitt der dreidimensionalen Struktur des Proteins auf Grundlage der Windungsstruktur-Information reproduziert, wobei die Windungsstruktur-Information aus dreidimensionaler Strukturinformation von Proteinen extrahiert wird, die bekannte dreidimensionale Strukturen aufweisen; und
ein Mittel zum Reproduzieren einer dreidimensionalen Struktur, das Reproduktions-Information erzeugt, in dem die dreidimensionale Struktur des Proteins durch Verwenden des reproduzierten Windungs-Abschnitts reproduziert wird.

2. Die Vorhcrsagevorrichtung für eine dreidimensionale Struktur eines Proteins nach Anspruch 1, wobei das Mittel zum Vorhersagen einer Windung umfasst,
ein Windungs-Information-Speichermittel, das die Windungsstruktur-Information in Verbindung mit der Sekundärstruktur und der Anzahl der die Windung ausbildenden Aminosäuren speichert, wobei die Windungsstruktur-Information zuvor aus der dreidimensionalen Strukturinformation gemäß der lokalen Sekundärstruktur-Information und der Anzahl der die Windung ausbildenden Aminosäuren extrahiert wird, und
ein Suchmittel, das die Windungsstruktur-Information durch Durchsuchen des Windungs-Information-Speichermittels auf Grundlage der Sekundärstruktur und der Anzahl der die Windung ausbildenden Aminosäuren gewinnt.

3. Die Vorhersagevorrichtung für eine dreidimensionale Struktur eines Proteins nach Anspruch 2, wobei wahrscheinliche Flächenwinkel, die durch Peptidebenen ausgebildet werden und die zuvor aus der dreidimensionalen Strukturinformation extrahiert werden, in dem Windungs-Information-Speichermittel entsprechend Sekundärstrukturen, zwischen denen die Windung auftritt, und der Anzahl der die Windung ausbildenden Aminosäuren gespeichert werden, und
das Suchmittel ein Flächenwinkel-Zuweisungsmittel enthält, das das Windungs-information-Speichermittel nach den Flächenwinkeln entsprechend den Sekundärstrukturen und der Anzahl der die Windung ausbildenden Aminosäuren durchsucht, und eine Zuweisung der Flächenwinkel durchführt.

4. Die Vorhersagevorrichtung für eine dreidimensionale Struktur eines Proteins nach Anspruch 3, wobei das Windungs-Information-Speichermittel entsprechend der Anzahl der die Windung ausbildenden Aminosäuren Flächenwinkel speichert, die wahrscheinlich sind, wenn die Windung zwischen β-Strängen auftritt und eine 180-Grad β-Strangwindung ist, die durch eine Windung eines vorbestimmten Winkels realisiert wird, der bei jeder der die Windung ausbildenden Aminosäuren auftritt.

5. Die Vorhersagevorrichtung für eine dreidimensionale Struktur eines Proteins nach Anspruch 3, wobei das Windungs-information-Speichermittel entsprechend der Anzahl der die Windung ausbildenden Aminosäuren Flächenwinkel speichert, die wahrscheinlich sind, wenn die Windung zwischen β-Strängen auftritt, und die erste und letzte der die Windung ausbildenden Aminosäuren eine 180-Grad β-Strangwindung ist, die auf eine solche Weise realisiert ist, dass andere die Windung ausbildenden Aminosäuren, die zwischen der ersten und letzten angeordnet sind, gebunden sind, um eine gerade Kette auszubilden.

6. Die Vorhersagevorrichtung für eine dreidimensionale Struktur eines Proteins nach Anspruch 1, wobei das Mittel zum Vorhersagen einer Windung ein Windungs-Richtung-Berechnungsmittel enthält, das die Richtung der Windung gemäß der Sekundärstruktur und, sofern erforderlich, der Anzahl der die Windung ausbildenden Aminosäuren ermittelt.

7. Die Vorhersagevorrichtung für eine dreidimensionale Struktur eines Proteins nach Anspruch 6, wobei, wenn die Windung zwischen β-Strängen auftritt, das Windungs-Richtung-Berechnungsmittel die Richtung der Windung danach ermittelt, ob die Anzahl der Aminosäuren, die einen β-Strang ausbilden und der Windung vorausgehen, ungeradzahlig oder geradzahlig ist.

8. Die vorhersagevorrichtung für eine dreidimensionale Struktur eines Proteins nach Anspruch 6, wobei, wenn die Windung zwischen α-Helices auftritt, das Windungs-Richtung-Berechnungsmittel die Richtung der Windung danach ermittelt, ob die Anzahl der die Windung ausbildenden Aminosäuren ungeradzahlig oder geradzahlig ist.

9. Ein computer-implementiertes Vorhersageverfahren für eine dreidimensionale Struktur eines Proteins zum Vorhersagen einer dreidimensionalen Struktur eines Proteins auf Grundlage einer Aminosäuresequenz, wobei das Protein aus den Aminosäuren ausgebildet ist, das **dadurch gekennzeichnet ist**, das es umfasst:
einen Schritt des vorherigcn Gewinnens und Speicherns von Windungsstruktur-Information über wahrscheinliche Windungen entsprechend lokaler Sekundärstruktur-Information und der Anzahl an Aminosäuren, die jede der wahrscheinlichen Windungen ausbilden, in einem vorherbestimmten Speichermittel, wobei die Windungsstruktur-Information aus dreidimensionaler Strukturinformation von Proteinen mit bekannten dreidimensionalen Strukturen extrahiert wird;
einen Schritt des Gewinnens der Aminosäuresequenz als eine Primärsequenz des Proteins und der lokalen Sekundärstruktur-Information, die aus der Aminosäuresequenz erhalten wird;
einen Schritt des Berechnens der Anzahl der eine Windung ausbildenden Aminosäuren auf Grundlage der lokalen Sekundärstruktur-Information;
einen Schritt des Extrahierens eines Abschnitts der Windungsstruktur-Information entsprechend der lokalen Sekundärstruktur-Information und der Anzahl der die Windung ausbildenden Aminosäuren, durch Durchsuchen des vorbestimmten Speichermittels nach dem Abschnitt der Windungsstruktur-Information auf Grundlage der lokalen Sekundärstruktur-Information und der Anzahl der die Windung ausbildenden Aminosäuren; und
einen Schritt des Erzeugens von Reproduktions-Information, bei dem die dreidimensionale Struktur des Proteins durch Verwenden eines Windungs-Abschnitts reproduziert wird, der auf Grundlage des extrahierten Abschnitts der Windungsstruktur-Information reproduziert wird.

10. Das Vorhersageverfahren für eine dreidimensionale Struktur eines Proteins nach Anspruch 9, wobei in dem Schritt des Extrahierens der Windungsstruktur-Information windungs-Abschnitte in dem Protein, dessen dreidimensionale Struktur vorhergesagt werden soll, auf Grundlage der lokalen Sekundärstruktur-Information gesucht werden, und ein Abschnitt der Windungsstruktur-Information entsprechend der Windung extrahiert wird, wenn die Windung ermittelt wird.

11. Das Vorhersageverfahren für eine dreidimensionale Struktur eines Proteins nach Anspruch 9, wobei in dem Schritt des Extrahierens der Windungsstruktur-Information das Protein, dessen dreidimensionale Struktur vorhergesagt werden soll, auf Grundlage der lokalen Sekundärstruktur-Information nach dem Windungs-Abschnitt durchsucht wird, auf den die Windungsstruktur-InformaLion angewandt wird, die gemäß der Sekundärstruktur-Information und der Anzahl der die Windung ausbildenden Aminosäuren extrahiert wurde.

12. Ein Programm zum Vorhersagen einer dreidimensionalen Struktur eines Proteins auf Grundlage einer Aminosäuresequenz, wobei das Protein aus den Aminosäuren ausgebildet ist, durch Verwenden eines Computers, das **dadurch gekennzeichnet ist, dass** es den Computer folgende Funktionen umfassen lässt:
ein Windungs-Ausbildungs-Abschnitt-Berechnungsmittel, das auf Grundlage der Aminosäuresequenz und lokaler Sekundärstruktur-Information die Anzahl an eine Windung ausbildenden Aminosäuren berechnet, wobei die Aminosäuresequenz als eine Primärsequenz des Proteins eingelesen wird, und die lokale Sekundärstruktur-Information aus der Aminosäuresequenz erhalten wird;
ein Mittel zum Vorhersagen einer Windung, das windungsstruktur-Information über eine wahrscheinliche Windung gemäß der lokalen Sekundärstruktur-Information und der Anzahl der die Windung ausbildenden Aminosäuren gewinnt, und das einen Windungs-Abschnitt der dreidimensionalen Struktur des Proteins auf Grundlage der Windungsstruktur-Information reproduziert, wobei die Windungsstruktur-Information aus dreidimensionaler Strukturinformation von Proteinen extrahiert wird, die bekannte dreidimensionale Strukturen aufweisen; und
ein Mittel zum Reproduzieren einer dreidimensionalen Struktur, das Reproduktions-Information erzeugt, in dem die dreidimensionale Struktur des Proteins durch Verwenden des reproduzierten Windungs-Abschnitts reproduziert wird.

13. Ein computer-lesbares Aufzeichnungsmedium, das ein Programm zum Vorhersagen einer dreidimensionalen Struktur eines Proteins auf Grundlage einer Aminosäuresequenz durch Verwenden eines Computers speichert, wobei das Protein aus den Aminosäuren ausgebildet ist, das **dadurch gekennzeichnet ist, dass** das Programm den Computer die Funktionen umfassen lässt:
ein Windungs-Ausbildungs-Abschnitt-Berechnungsmittel, das auf Grundlage der Aminosäuresequenz und lokaler Sekundärstruktur-Information die Anzahl an eine Windung ausbildenden Aminosäuren berechnet, wobei die Aminosäuresequenz als eine Primärsequenz des Proteins eingelesen wird, und die lokale Sekundärstruktur-Information aus der Aminosäuresequenz erhalten wird;
ein Mittel zum Vorhersagen einer Windung, das Windungsstruktur-Information über eine wahrscheinliche Windung gemäß der lokalen Sekundärstruktur-Information und der Anzahl der die Windung ausbildenden Aminosäuren gewinnt, und das einen Windungs-Abschnitt der dreidimensionalen Struktur des Proteins auf Grundlage der Windungsstruktur-Information reproduziert, wobei die Windungsstruktur-Information aus dreidimensionaler Strukturinformation von Proteinen extrahiert wird, die bekannte dreidimensionale Strukturen aufweisen; und
ein Mittel zum Reproduzieren einer dreidimensionalen Struktur, das Reproduktions-Information erzeugt, in dem die dreidimensionale Struktur des Proteins durch verwenden des reproduzierten Windungs-Abschnitts reproduziert wird.

## Revendications

1. Appareil de prédiction de la structure tridimensionnelle d'une protéine pour prédire, en se fondant sur une séquence d'acides aminés, une structure tridimensionnelle d'une protéine formée des acides aminés, **caractérisé en ce qu'**il comprend :
un moyen de calcul de partie formant des enroulements qui calcule le nombre d'acides aminés formant un enroulement, en se fondant sur ladite séquence des acides aminés et sur des informations locales de structure secondaire d'une structure secondaire, la séquence des acides aminés étant lue comme une séquence primaire de ladite protéine, et les informations locales de structure secondaire étant obtenues à partir de la séquence des acides aminés ;
un moyen de prédiction d'enroulement qui acquiert les informations de structure d'enroulement sur un enroulement probable, selon lesdites informations locales de structure secondaire et ledit nombre des acides aminés formant ledit enroulement, et qui reproduit une partie enroulée de la structure tridimensionnelle de ladite protéine en se fondant sur les informations de structure d'enroulement, les informations de structure d'enroulement étant extraites des informations de structure tridimensionnelle de protéines ayant des structures tridimensionnelles connues ; et
un moyen de reproduction de structure tridimensionnelle qui génère des informations de reproduction, la structure tridimensionnelle de ladite protéine étant reproduite en utilisant la partie enroulée reproduite.

2. Appareil de prédiction de la structure tridimensionnelle d'une protéine selon la revendication 1, dans lequel ledit moyen de prédiction d'enroulement comprend
un moyen d'enregistrement des informations d'enroulement qui enregistre lesdites informations de structure d'enroulement en association avec ladite structure secondaire et ledit nombre des acides aminés formant ledit enroulement, les informations de structure d'enroulement étant extraites par avance à partir desdites informations de structure tridimensionnelles selon les informations locales de structure secondaire et le nombre des acides aminés formant l'enroulement, et
un moyen de recherche qui acquiert lesdites informations de structure d'enroulement en effectuant une recherche dans ledit moyen d'enregistrement d'informations d'enroulement en se fondant sur ladite structure secondaire et le nombre des acides aminés formant ledit enroulement.

3. Appareil de prédiction de la structure tridimensionnelle d'une protéine selon la revendication 2, les angles dièdres probables qui sont formés par les plans peptidiques et extraits par avance desdites informations de structure tridimensionnelle étant enregistrés dans ledit moyen d'enregistrement d'informations d'enroulement en correspondance avec les structures secondaires entre lesquelles ledit enroulement apparaît et ledit nombre des acides aminés formant l'enroulement, et
ledit moyen de recherche comprenant un moyen d'attribution d'angle dièdre qui effectue une recherche dans ledit moyen d'enregistrement d'informations d'enroulement, lesdits angles dièdres correspondant aux dites structures secondaires et au dit nombre des acides aminés formant l'enroulement, et qui réalise l'attribution desdits angles dièdres.

4. Appareil de prédiction de la structure tridimensionnelle d'une protéine selon la revendication 3, ledit moyen d'enregistrement d'informations d'enroulement enregistrant, en correspondance avec ledit nombre des acides aminés formant l'enroulement, les angles dièdres qui sont probables lorsque ledit enroulement apparaît entre des brins β et est un enroulement de brin β à 180 degrés réalisé par un enroulement d'un angle prédéterminé survenant au niveau de chacun desdits acides aminés formant l'enroulement.

5. Appareil de prédiction de la structure tridimensionnelle d'une protéine selon la revendication 3, ledit moyen d'enregistrement des informations d'enroulement enregistrant, en correspondance avec ledit nombre des acides aminés formant l'enroulement, des angles dièdres qui sont probables lorsque ledit enroulement qui apparaît entre des brins β et lesdits premier et dernier desdits acides aminés formant l'enroulement est un enroulement de brin β à 180 degrés réalisé de telle manière que les autres desdits acides aminés formant l'enroulement qui se trouvent entre le premier et le dernier sont liés pour former une chaîne droite.

6. Appareil de prédiction de la structure tridimensionnelle d'une protéine selon la revendication 1, ledit moyen de prédiction d'enroulement comprenant un moyen de calcul de direction d'enroulement qui détermine la direction de l'enroulement selon ladite structure secondaire et, lorsque c'est nécessaire, ledit nombre des acides aminés formant l'enroulement.

7. Appareil de prédiction de la structure tridimensionnelle d'une protéine selon la revendication 6, dans lequel, lorsque ledit enroulement apparaît entre des brins β, ledit moyen de calcul de direction d'enroulement détermine la direction de l'enroulement selon si le nombre des acides aminés formant un brin β et précédant ledit enroulement est impair ou pair.

8. Appareil de prédiction de la structure tridimensionnelle d'une protéine selon la revendication 6, dans lequel, lorsque ledit enroulement apparaît entre des hélices α, ledit moyen de calcul de direction d'enroulement détermine la direction de l'enroulement selon si le nombre des acides aminés formant ledit enroulement est impair ou pair.

9. Procédé informatisé de prédiction de la structure tridimensionnelle d'une protéine pour prédire, en se fondant sur une séquence d'acides aminés, une structure tridimensionnelle d'une protéine formée des acides aminés, **caractérisé en ce qu'**il comprend :
une étape consistant à acquérir par avance et à enregistrer dans un moyen d'enregistrement prédéterminé les informations de structure d'enroulement correspondant aux informations locales de structure secondaire et au nombre d'acides aminés formant chacun des enroulements probables, les informations de structure d'enroulement étant extraites d'informations de structure tridimensionnelle de protéines ayant des structures tridimensionnelles connues ;
une étape consistant à acquérir ladite séquence des acides aminés comme séquence primaire de ladite protéine et lesdites informations locales de structure secondaire qui sont obtenues à partir de la séquence des acides aminés ;
une étape consistant à calculer le nombre des acides aminés formant un enroulement, en se fondant sur lesdites informations locales de structure secondaire ;
une étape consistant à extraire une partie desdites informations de structure d'enroulement correspondant auxdites informations locales de structure secondaire et au dit nombre des acides aminés formant ledit enroulement, en recherchant dans ledit moyen d'enregistrement prédéterminé la partie des informations de structure d'enroulement en se fondant sur les informations locales de structure secondaire et le nombre des acides aminés formant ledit enroulement; et
une étape consistant à générer des informations de reproduction dans lesquelles la structure tridimensionnelle de ladite protéine est reproduite en utilisant une partie d'enroulement reproduite en se fondant sur la partie extraite des informations de structure d'enroulement.

10. Procédé de prédiction de la structure tridimensionnelle d'une protéine selon la revendication 9, dans lequel, au cours de ladite étape consistant à extraire lesdites informations de structure d'enroulement, les parties enroulées dans ladite protéine dont la structure tridimensionnelle doit être prédite sont recherchées en se fondant sur lesdites informations locales de structure secondaire, et une partie des informations de structure d'enroulement correspondant au dit enroulement est extraite lorsque ledit enroulement est détecté.

11. Procédé de prédiction de la structure tridimensionnelle d'une protéine selon la revendication 9, dans lequel, au cours de ladite étape consistant à extraire lesdites informations de structure d'enroulement, on recherche dans ladite protéine dont la structure tridimensionnelle doit être prédite, en se fondant sur lesdites informations locales de structure secondaire, ladite partie enroulée à laquelle lesdites informations de structure d'enroulement extraites selon lesdites informations de structure secondaire et le nombre des acides aminés formant l'enroulement sont appliquées.

12. Programme pour prédire, en se fondant sur une séquence d'acides aminés, une structure tridimensionnelle d'une protéine formée des acides aminés, en utilisant un ordinateur, **caractérisé en ce que** ledit programme confère au dit ordinateur les fonctions suivantes :
un moyen de calcul de partie formant des enroulements qui calcule le nombre d'acides aminés formant un enroulement, en se fondant sur ladite séquence des acides aminés et les informations locales de structure secondaire, la séquence des acides aminés étant lue comme une séquence primaire de ladite protéine, et les informations locales de structure secondaire étant obtenues à partir de la séquence des acides aminés ;
un moyen de prédiction d'enroulement qui acquiert les informations de structure d'enroulement sur un enroulement probable, selon lesdites informations locales de structure secondaire et ledit nombre des acides aminés formant ledit enroulement, et qui reproduit une partie d'enroulement de la structure tridimensionnelle de ladite protéine en se fondant sur les informations de structure d'enroulement, les informations de structure d'enroulement étant extraites des informations de structure tridimensionnelle de protéines ayant des structures tridimensionnelles connues ; et
un moyen de reproduction de structure tridimensionnelle qui génère des informations de reproduction dans lesquelles la structure tridimensionnelle de ladite protéine est reproduite en utilisant la partie d'enroulement reproduite.

13. Support d'enregistrement informatisé enregistrant un programme pour prédire, en se fondant sur une séquence d'acides aminés, une structure tridimensionnelle d'une protéine formée des acides aminés, en utilisant un ordinateur, **caractérisé en ce que** ledit ordinateur comprend les fonctions suivantes :
un moyen de calcul de partie formant des enroulements qui calcule le nombre d'acides aminés formant un enroulement, en se fondant sur ladite séquence des acides aminés et les informations locales de structure secondaire, la séquence des acides aminés étant lue comme une séquence primaire de ladite protéine, et les informations locales de structure secondaire étant obtenues à partir de la séquence des acides aminés ;
un moyen de prédiction d'enroulement qui acquiert des informations de structure d'enroulement sur un enroulement probable, en fonction desdites informations locales de structure secondaire et dudit nombre des acides aminés formant ledit enroulement, et qui reproduit une partie enroulée de la structure tridimensionnelle de ladite protéine fondée sur les informations de structure d'enroulement, les informations de structure d'enroulement étant extraites des informations de structure tridimensionnelle de protéines ayant des structure tridimensionnelles connues ; et
un moyen de reproduction de structure tridimensionnelle qui génère des informations de reproduction dans lequel la structure tridimensionnelle de ladite protéine est reproduite en utilisant la partie d'enroulement reproduite.


### SEQUENCE LISTING

<110> FUJITSU LIMITED
<120> APPARATUS AND METHOD FOR PREDICTING THREE-DIMENSIONAL STRUCTURE OF PROTEIN
<130> FUP-1468P
<140> PCT/JP02/12942
   <141> 2002-12-10
<150> JP 2001-375857
   <151> 2001-12-10
<160> 1
<170> Patentln version 3.1
<210> 1
   <211> 228
   <212> PRT
   <213> Artificial Sequence
<220>
   <223> Inventor: Sakai, Kohta
<220>
   <221> TURN
   <222> (29).. (32)
   <223> a turn which appears in an amino-acid sequence
<220>
   <221> TURN
   <222> (55).. (61)
   <223> a turn which appears in an amino-acid sequence
<220>
   <221> TURN
   <222> (68).. (69)
   <223> a turn which appears in an amino-acid sequence
<400> 1
   Thr Gly Arg Pro Glu Trp Ile Trp Leu Ala Leu Gly Thr Ala Leu Met
   1               5                   10                  15
   Gly Leu Gly Thr Leu Tyr Phe Leu Val Lys Gly Met Gly Val Ser Asp
               20                  25                  30
   Pro Asp Ala Lys Lys Phe Tyr Ala Ile Thr Thr Leu Val Pro Ala Ile
   35 40 45
   Ala Phe Thr Met Tyr Leu Ser Met Leu Leu Gly Tyr Gly Leu Thr Met
   50 55 60
   Val Pro Phe Gly Gly Glu Gln Asn Pro Ile Tyr Trp Ala Arg Tyr Ala
   65 70 75 80
   Asp Trp Leu Phe Thr Thr Pro Leu Leu Leu Leu Asp Leu Ala Leu Leu
   85 90 95
   Val Asp Ala Asp Gln Gly Thr Ile Leu Ala Leu Val Gly Ala Asp Gly
   100 105 110
   Ile Met Ile Gly Thr Gly Leu Val Gly Ala Leu Thr Lys Val Tyr Ser
   115 120 125
   Tyr Arg Phe Val Trp Trp Ala Ile Ser Thr Ala Ala Met Leu Tyr Ile
   130 135 140
   Leu Tyr Val Leu Phe Phe Gly Phe Thr Ser Lys Ala Glu Ser Met Arg
   145 150 155 160
   Pro Glu Val Ala Ser Thr Phe Lys Val Leu Arg Asn Val Thr Val Val
   165 170 175
   Leu Trp Ser Ala Tyr Pro Val Val Trp Leu Ile Gly Ser Glu Gly Ala
   180 185 190
   Gly Ile Val Pro Leu Asn Ile Glu Thr Leu Leu Phe Met Val Leu Asp
   195 200 205
   Val Ser Ala Lys Val Gly Phe Gly Leu Ile Leu Leu Arg Ser Arg Ala
   210 215 220
   Ile Phe Gly Glu
   225